# EUROPEAN PATENT APPLICATION

(11) **EP 4 768 588 A1**
(43) Date of publication of application: **01.07.2026**
(21) Application number: 26150091.2
(22) Date of filing: 02.01.2026
(51) Int. Cl.: C12N 15/113

(54) **MIR-204-TARGETING OLIGONUCLEOTIDE AND COMPOSITION THEREOF FOR THE IMPROVEMENT, PREVENTION OR TREATMENT OF OSTEOARTHRITIS**

(30) Priority: 27.12.2024 KR 20240198889; 09.12.2025 KR 20250194238; 24.12.2025 EP 25227249
(71) Applicant: Liflex Science Inc., Seoul 08826 (KR); Seoul National University R&DB Foundation, Seoul 08826 (KR)
(72) Inventor: KIM, Jin-Hong, 06320 Seoul (KR); KANG, Donghyun, 08826 Seoul (KR); YONG, Sangmin, 08789 Seoul (KR); KIM, Soy, 08788 Seoul (KR); JEONG, Sehan, 08788 Seoul (KR); LEE, Ji-Eun, 13924 Anyang-si (KR); CHO, Yongsik, 03306 Seoul (KR); KIM, Hyeonkyeong, 08787 Seoul (KR); LEE, Seung Hyun, 08838 Seoul (KR)
(74) Representative: V.O.

(57) **Abstract**

The invention relates to the field of medicine, in particular to means and methods for the improvement, prevention or treatment of osteoarthritis. Provided is an oligonucleotide specifically hybridizing with miR-204 and a composition for ameliorating, preventing, or treating osteoarthritis, including the same. The oligonucleotide includes 20 to 22 linked nucleotides and consists of a first region, a second region, and a third region in order from a 5' end to a 3' end, wherein the first region includes three consecutively connected nucleotides, each complementary to a tail region of miR-204 and including a constrained sugar modification, the second region includes three connected nucleotides connected, the nucleotides included in the second region each independently being nucleotides including a constrained sugar modification or a non-constrained 2'-substituted sugar modification, and the third region includes a plurality of connected nucleotides, the nucleotides included in the third region each independently being nucleotides including a non-constrained 2'-substituted sugar modification, and wherein the oligonucleotide includes 30% or less of nucleotides including a constrained sugar modification based on a total number of nucleotides.

## Description

### [Technical Field]

This application claims priority to and the benefit of Korean Patent Application No. 10-2024-0198889, filed on December 27, 2024, the disclosure of which is incorporated herein by reference in its entirety. This application also claims priority to and the benefit of Korean Patent Application No. 10-2025-0198889, filed on December 9, 2025, the disclosure of which is incorporated herein by reference in its entirety.

Disclosed herein are an oligonucleotide specifically hybridizing with miR-204, and a composition for ameliorating, preventing, or treating osteoarthritis (OA), including the same.

Description of Government-Supported Research Project
[National Research and Development Project Supporting This Invention]
[Project Identification Number] 2420003227
[Project Number] RS-2024-00437503
[Ministry] Ministry of SMEs and Startups
[Project Management (Specialized) Agency] Korea Technology Information Promotion Agency for SMEs
[Research Project Name] Startup Growth Technology Development
[Research Project Name] Development of a Next-Generation Osteoarthritis Treatment Based on MicroRNA Degraders
[Project Implementing Agency] LIFLEX SCIENCE INC.
[Research Period] July 1, 2024 to June 30, 2027

### [Background Art]

Osteoarthritis (OA) is a disease that affects 4.18 million people in Korea alone as of 2022, with the number of patients worldwide having increased by 48% between 1990 and 2019. The demand for OA treatment is rapidly increasing due to the increase in patients resulting from aging and increased interest in quality of life. Cartilage is a tissue that undergoes progressive degeneration when damaged by various etiological risk factors such as aging, mechanical overload, obesity, and metabolic diseases, and OA is known to develop as a result of cartilage degeneration. OA is characterized by pathological changes including cartilage degeneration, subchondral bone sclerosis, osteophyte development, and synovial inflammation.

Existing OA treatment research has been limited to slowing cartilage degeneration by blocking the "degradation" mechanism of cartilage extracellular matrix, and OA treatment strategies rely on prescribing anti-inflammatory drugs or arthroplasty due to the absence of therapeutics that fundamentally ameliorate the disease.

miR-204 is a type of microRNA, specifically referring to miR-204-5p. miR-204 is a microRNA that is significantly increased in expression in OA cartilage of various species, simultaneously inhibits multiple sulfated glycosaminoglycan (sGAG) synthesis enzymes that constitute the cartilage matrix, and contributes to senescence-associated secretory phenotype (SASP) formation, thereby exacerbating OA. Since the therapeutic effect of miR-204 inhibition for OA treatment is known, it is now time to develop therapeutic drugs that effectively "degrade" miR-204 with minimal side effects. However, despite the importance of reducing sGAG synthesis and SASP formation by miR-204 in the pathogenesis of OA, no effective therapeutic agent capable of specifically binding to miR-204 to restore cartilage matrix synthesis or suppress inflammation-related factors has been developed to date.

The present inventors identified miR-204 as a biomarker for ameliorating, preventing, or treating osteoarthritis (OA) and conducted research on nucleic acid therapeutics targeting the same, thereby confirming that an oligonucleotide specifically hybridizing with miR-204 exhibits excellent OA amelioration, prevention, or treatment effects by degrading miR-204, when a nucleotide at a specific position among the nucleotides constituting the oligonucleotide includes a constrained nucleic acid modification or a non-constrained 2'-substituted sugar modification.

Accordingly, in one aspect, an object of the present invention is to provide an oligonucleotide specifically hybridizing with miR-204 and exhibits excellent degradation activity against miR-204.

In another aspect, an object of the present invention is to provide a composition for ameliorating, preventing, or treating OA, including the oligonucleotide.

One aspect of the present invention provides an oligonucleotide specifically hybridizing with miR-204, wherein the oligonucleotide includes 20 to 22 linked nucleotides and consists of a first region, a second region, and a third region in order from a 5' end to a 3' end, wherein the first region includes three consecutively connected nucleotides, each complementary to a tail region of miR-204 and including a constrained sugar modification, the second region includes three connected nucleotides connected, the nucleotides included in the second region each independently being nucleotides including a constrained sugar modification or a non-constrained 2'-substituted sugar modification, and the third region includes a plurality of connected nucleotides, the nucleotides included in the third region each independently being nucleotides including a non-constrained 2'-substituted sugar modification, and wherein the oligonucleotide includes 30% or less of nucleotides including a constrained sugar modification based on a total number of nucleotides.

Another aspect of the present invention provides a composition for ameliorating, preventing, or treating osteoarthritis (OA), including the oligonucleotide.

The oligonucleotide according to one aspect of the present invention is an antisense oligonucleotide that reduces the expression of miR-204 by inducing its degradation, and by comparing and verifying various chemical modifications including sugar modifications, length adjustment, and selection of the number and positions of constrained sugar modifications, the oligonucleotide is designed to have a structure optimized for miR-204 inhibition efficacy and formulation stability,

Accordingly, the antisense oligonucleotide of the present invention effectively blocks the pathophysiological processes of osteoarthritis (OA), such as decreased cartilage matrix synthesis and increased inflammatory response, and thus is useful for pain relief and prevention or treatment of OA.

In addition, the oligonucleotide according to one aspect of the present invention exhibits excellent delivery to chondrocytes and stable residence in tissue, thereby providing a structural advantage capable of achieving long-term sustained therapeutic effects.

The oligonucleotide according to one aspect of the present invention has low potential for inducing immunogenicity and low toxicity, thereby providing safety suitable for use as a pharmaceutical composition.

The oligonucleotide according to one aspect of the present invention has been confirmed to effectively inhibit miR-204 not only in human chondrocytes but also in canine chondrocytes, and furthermore, since the nucleotide sequence of miR-204 is conserved among multiple species including humans, dogs, and monkeys, the oligonucleotide can be utilized as a therapeutic agent and/or composition for feed additives in various animal species.

Since the sequence of the target miR-204 is conserved across species, the oligonucleotide according to one aspect of the present invention can be utilized not only in human-derived chondrocytes or humans, but also in chondrocytes of other species (e.g., dogs, monkeys, etc.) or in other species.

### LEGEND TO THE FIGURES

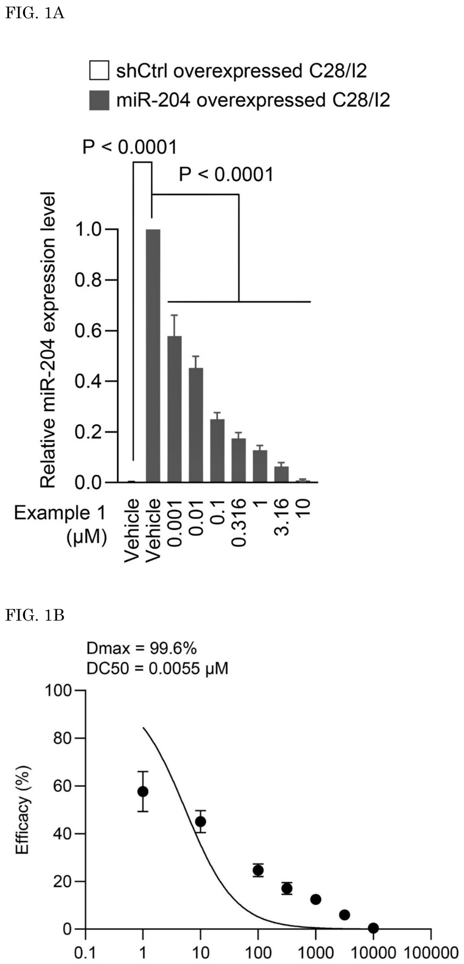

FIG. 1 shows an experimental example of the present invention and illustrates the resulting of measuring miR-204 degradation effect by qRT-PCR in the C28/I2 chondrocyte cell line by treatment of Example 1 according to Experimental Example 1.

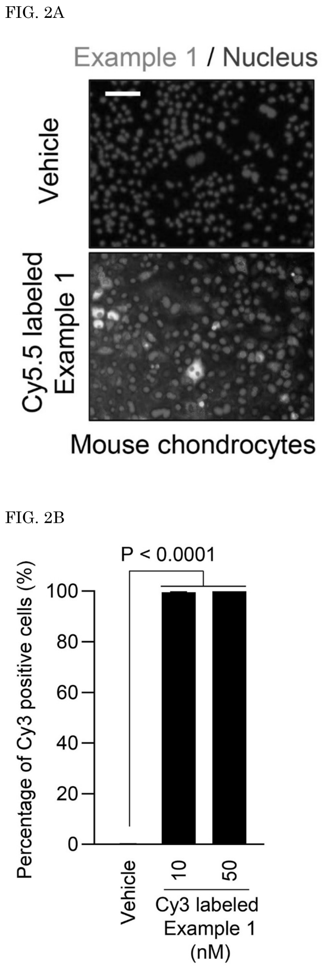

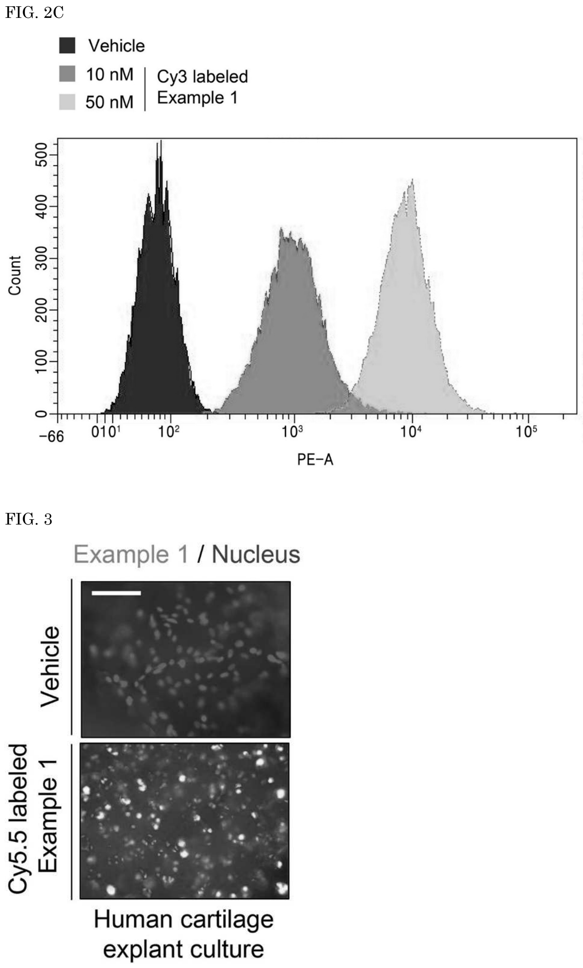

FIG. 2 shows the results of confirming the delivery efficiency of Example 1 into mouse-derived chondrocytes according to an experimental example of the present invention. FIG. 2A shows fluorescence microscopy images of fluorescently labeled Example 1 accumulated in mouse-derived chondrocytes, and FIGS. 2B and 2C show the results of quantifying the proportion of Cy3 fluorescently labeled Example 1-positive cells confirmed through flow cytometry analysis.
FIG. 3 shows fluorescence microscopy images confirming the delivery efficiency of Example 1 in cartilage tissue derived from osteoarthritis (OA) patients using fluorescent-labeled Example 1 according to an experimental example of the present invention.

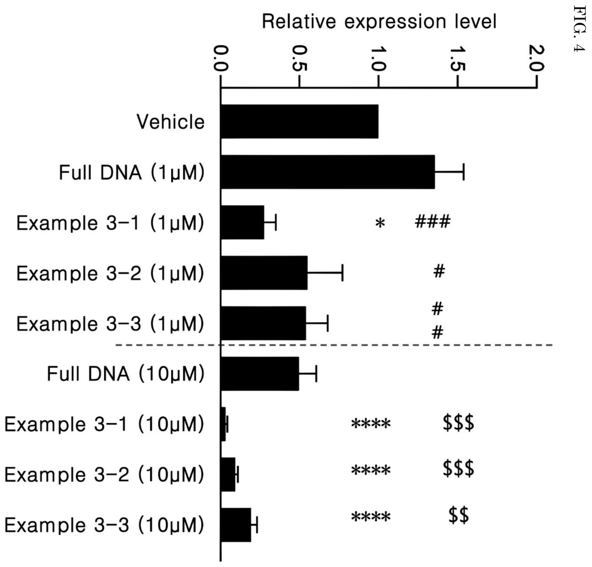

FIG. 4 shows a graph illustrating the results of measuring the expression level of miR-204 by qRT-PCR when treated with the full DNA or antisense oligonucleotides (ASOs) including mismatches within the sequence (Examples 3-1 to 3-3) according to Experimental Example 4, which is an experimental example of the present invention.

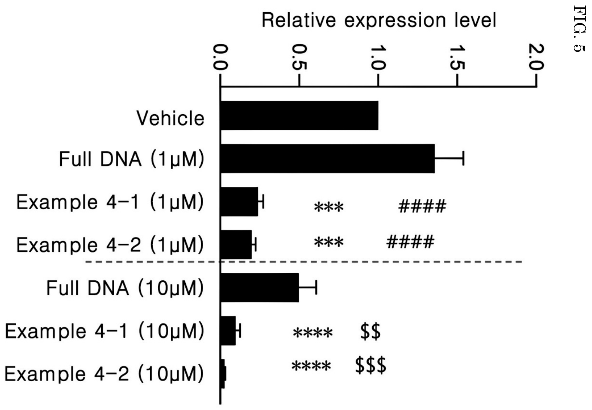

FIG. 5 shows a graph illustrating the results of measuring the expression level of miR-204 by qRT-PCR when treated with the full DNA or ASOs (Examples 4-1 and 4-2) having one or two bases truncated from the 5' end based on a microRNA according to Experimental Example 5 of the present invention.

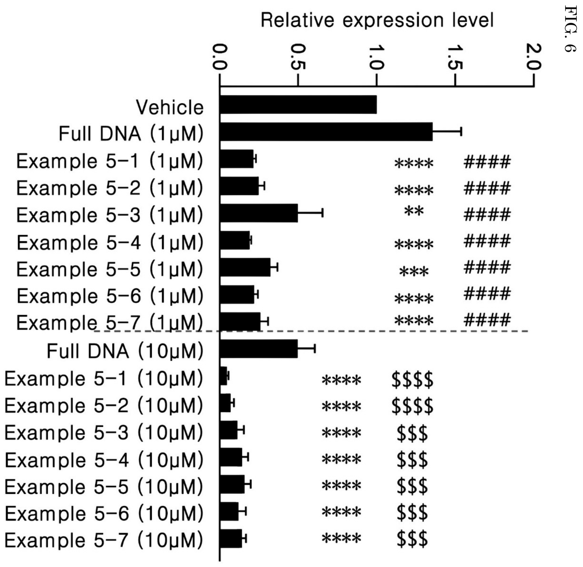

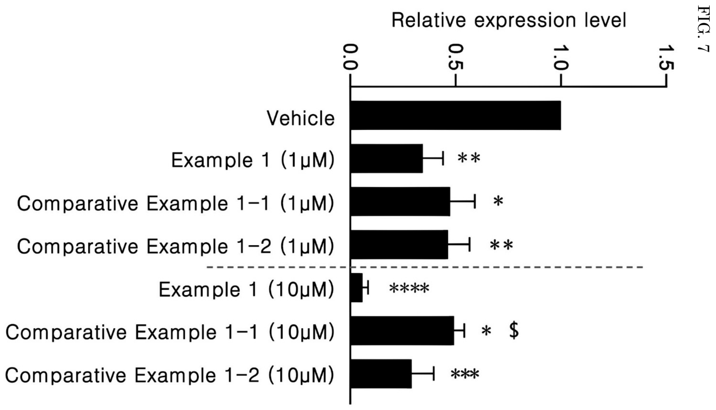

FIGS. 6 and 7 show graphs illustrating the experimental results of measuring miR-204 expression levels by qRT-PCR to determine whether there is a difference in miR-204 degradation ability depending on whether each nucleotide included in a first region and a second region of ASOs specific to miR-204 according to one embodiment of the present invention includes a constrained sugar modification (Full DNA, Examples 5-1 to 5-7, and Comparative Examples 1-1 and 1-2).

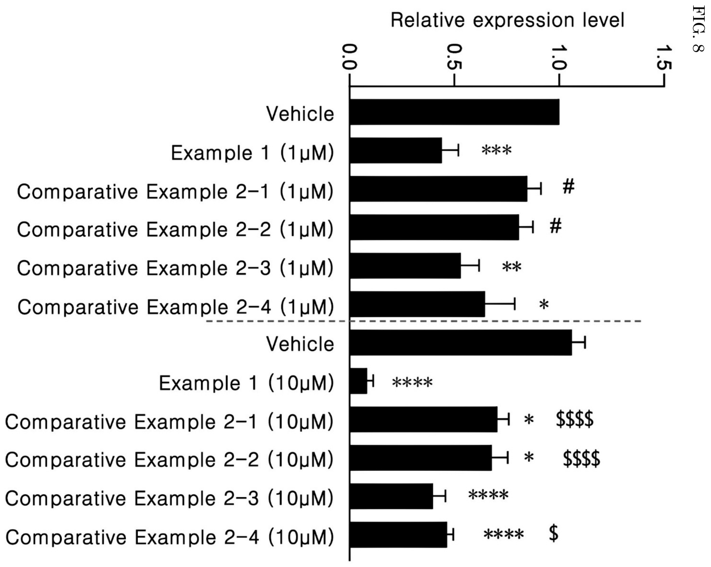

FIG. 8 shows a graph illustrating the experimental results of measuring miR-204 expression levels by qRT-PCR to determine miR-204 degradation ability when ASOs specific to miR-204 includes a locked nucleic acid (LNA) at one or more positions among position 1 to position 6 in the direction from a 3' end to a 5' end (Example 1, Comparative Examples 2-1 to 2-4) as a comparative example of the present invention.

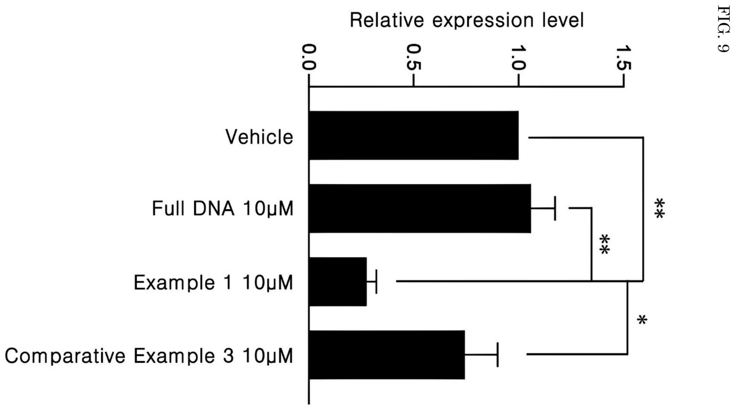

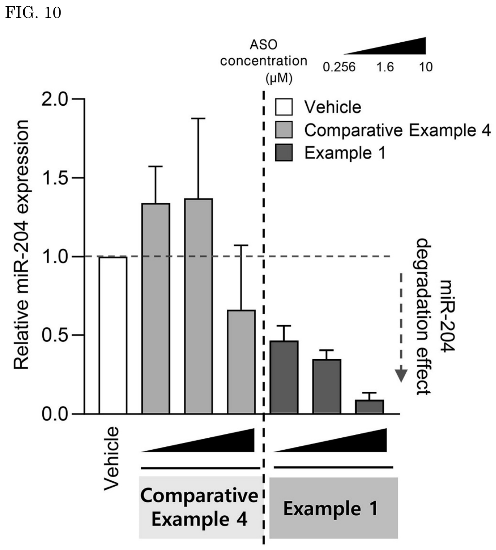

FIGS. 9 and 10 show graphs illustrating the experimental results of measuring miR-204 expression levels by qRT-PCR to determine miR-204 degradation ability when the ASOs specific to miR-204 randomly includes an LNA and/or has a shorter length than ASOs of one embodiment of the present invention (Comparative Examples 3 and 4), as comparative examples of the present invention compared to Full DNA or Example 1, which is an ASO according to one embodiment of the present invention.

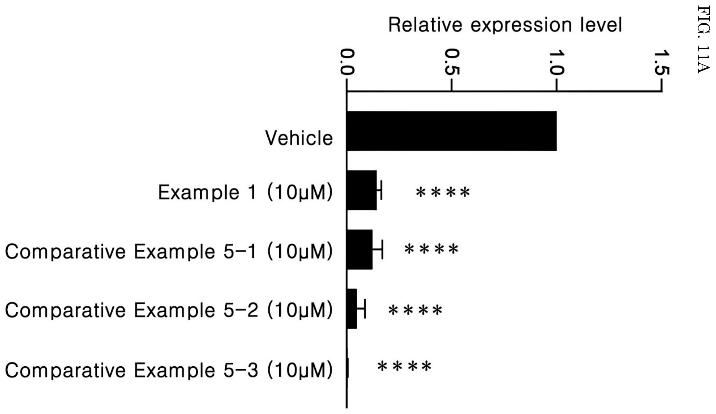

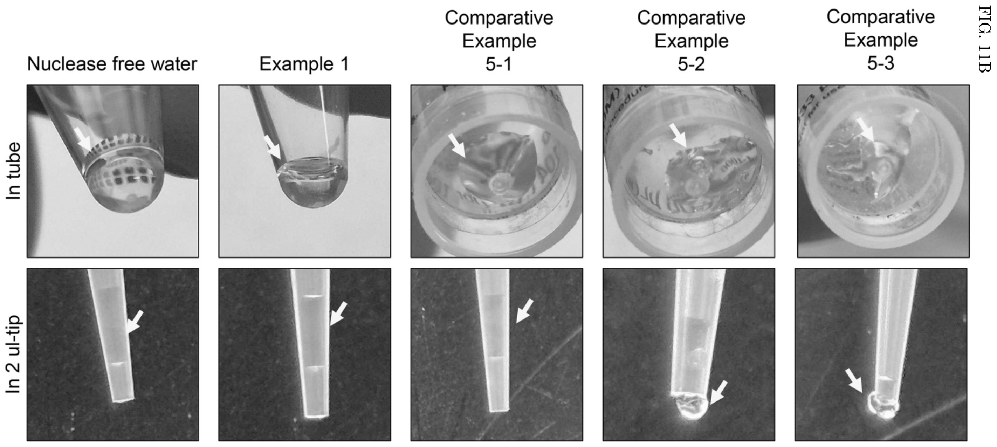

FIGS. 11A and 11B show a graph (FIG. 11A) illustrating the experimental results of measuring miR-204 expression levels by qRT-PCR to determine miR-204 degradation ability when ASOs specific to miR-204 include 15 or more LNAs (Comparative Examples 5-1 to 5-3), and photographs (FIG. 11B) taken to confirm formulation stability when the ASOs are dissolved, as comparative examples of the present invention.

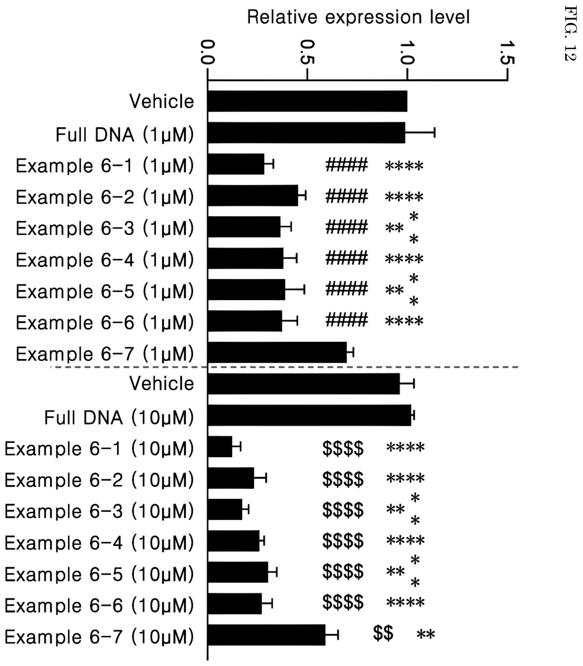

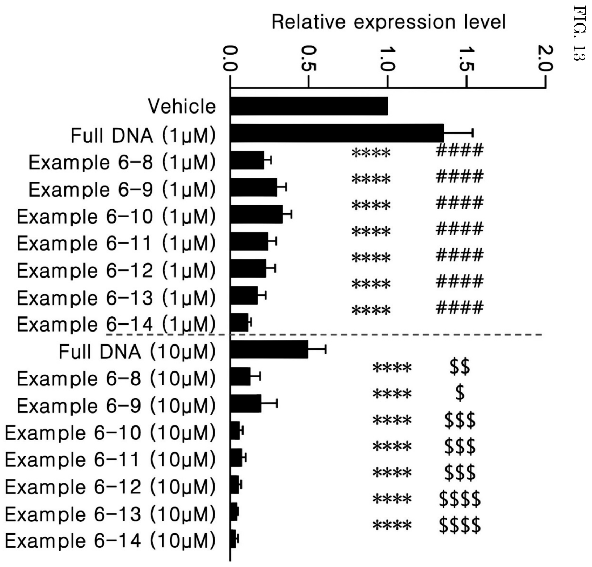

FIGS. 12 and 13 show graphs illustrating the experimental results of measuring miR-204 expression levels by qRT-PCR to determine miR-204 degradation ability when ASOs specific to miR-204 according to an embodiment of the present invention include one or more nucleotides including 2'-O-methoxyethyl (MOE) or 2'-O-methyl (OME) instead of LNA in the second region (Examples 6-1 to 6-14).

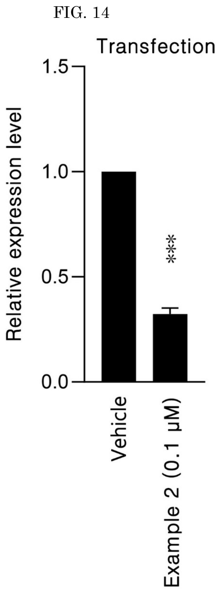

FIG. 14 shows a graph illustrating the experimental results of measuring miR-204 expression levels by qRT-PCR to determine miR-204 degradation ability when the ASOs specific to miR-204 according to an embodiment of the present invention includes a phosphodiester (PO) bond (backbone) instead of a phosphorothioate (PS) bond (backbone) (Example 2).

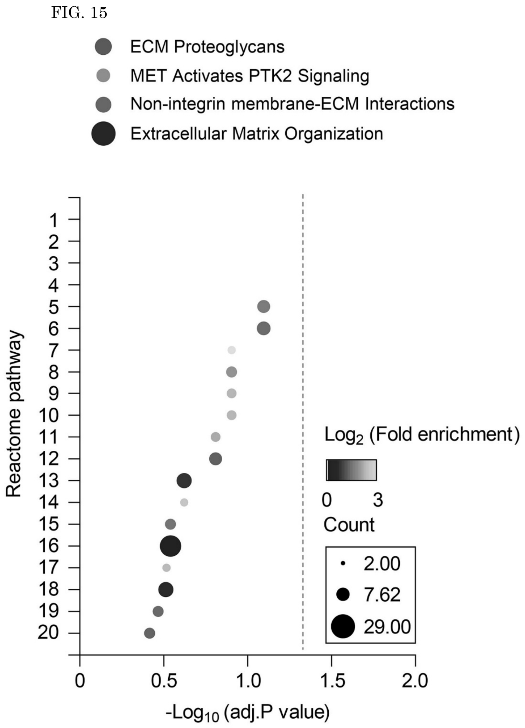

FIG. 15 shows a graph illustrating pathway enrichment results in a bubble plot format based on transcriptome analysis results between an Example 1 treatment group and a vehicle control group according to an embodiment of the present invention. In FIG. 15, the size of the dots represents the number of genes included in the corresponding pathway, and the color represents the fold enrichment value.

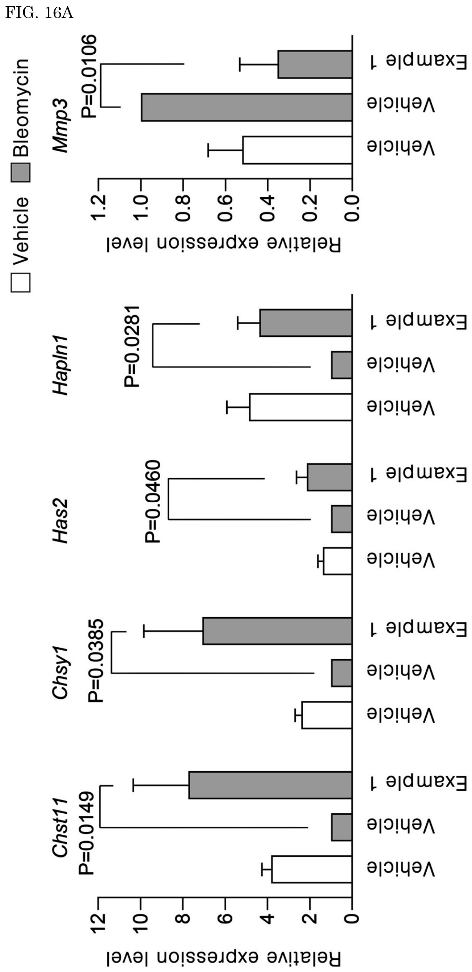

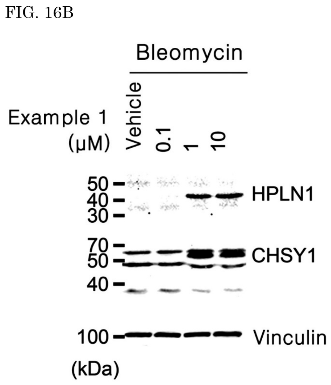

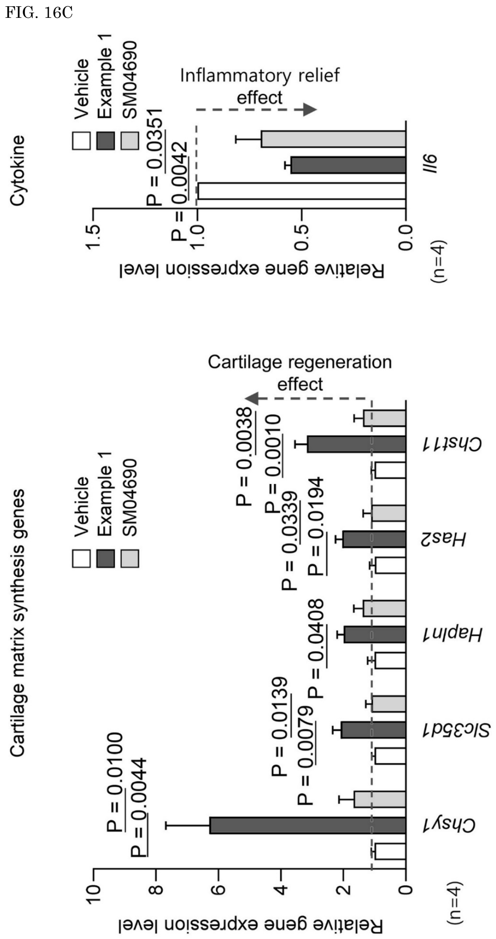

FIGS. 16A to 16C show diagrams illustrating changes in gene and protein expression related to ECM composition or inflammation induction according to treatment of Example 1 as an embodiment of the present invention in mouse-derived chondrocytes. FIG. 16A shows a graph illustrating the results of measuring mRNA expression of sulfated glycosaminoglycan (sGAG) biosynthesis pathway-related enzymes by qRT-PCR in chondrocytes with bleomycin-induced damage. FIG. 16B shows a band image illustrating the results of Western blot analysis of sGAG synthesis-related protein expression. FIG. 16C shows a graph illustrating the results of measuring changes in ECM biosynthesis enzyme and inflammation-related gene expression by qRT-PCR according to the treatment with Example 1 and SM04690 under inflammatory cytokine IL-1β treatment conditions.

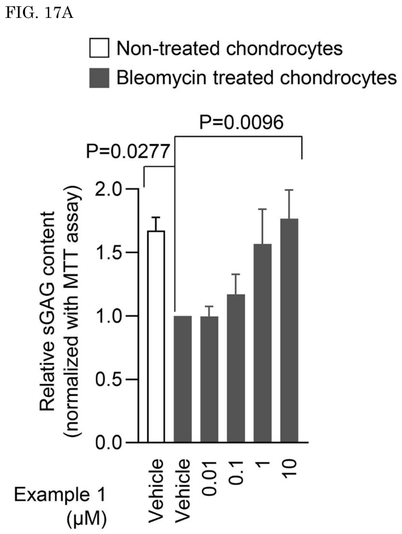

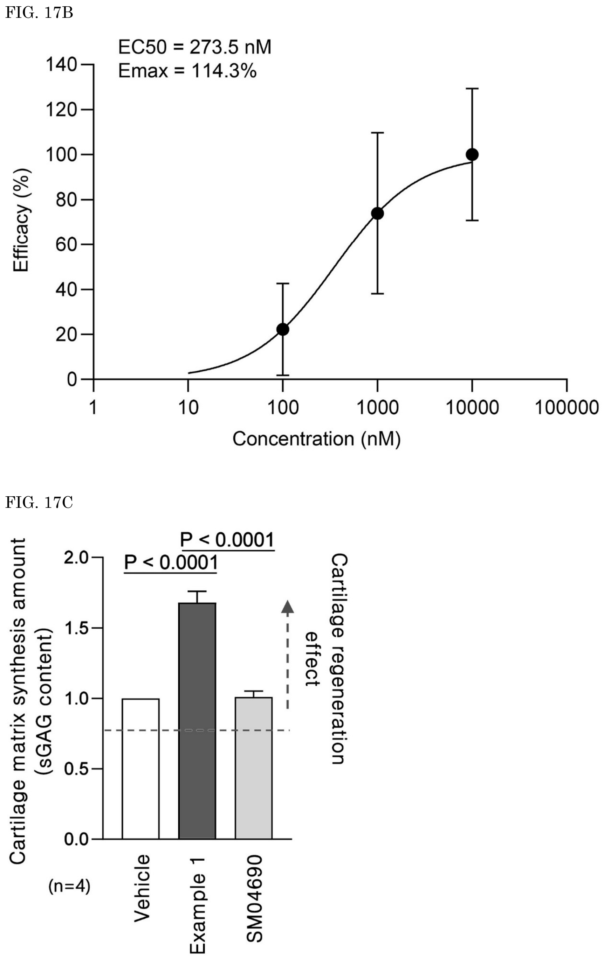

FIGS. 17A to 17C show changes in sGAG content in chondrocytes according to treatment with Example 1 according to an embodiment of the present invention. FIG. 17A shows a graph illustrating changes in sGAG content according to treatment concentration of Example 1 in chondrocytes with bleomycin-induced damage. FIG. 17B shows a graph illustrating the results of Example 1 from FIG. 17A replotted on a logarithmic scale in the form of a concentration-response curve, displaying the calculated maximum effect (Eₘₐₓ) and half-maximal effective concentration (EC50) values. FIG. 17C shows a graph illustrating changes in sGAG content according to treatment with Example 1 and SM04690 under inflammatory cytokine IL-1β treatment conditions.

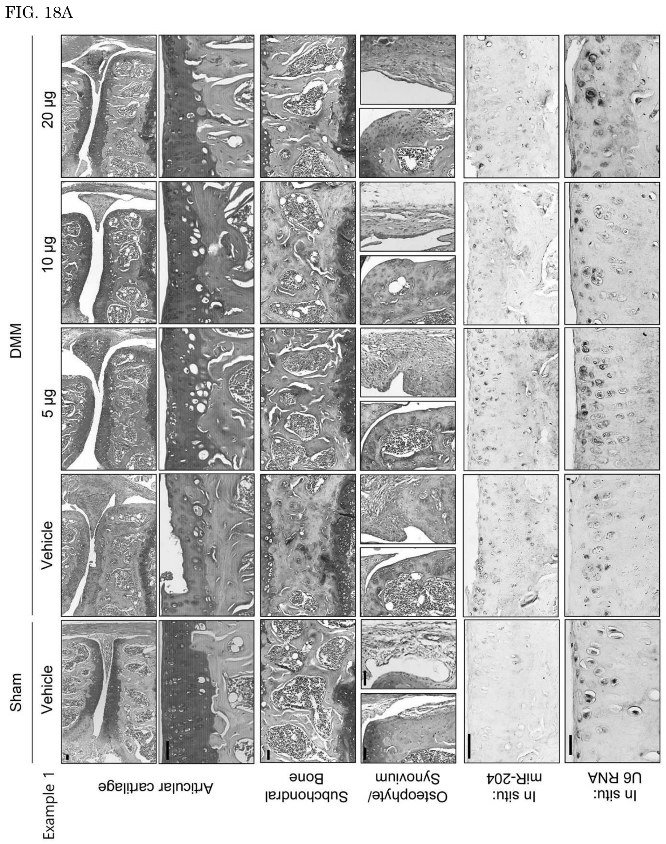

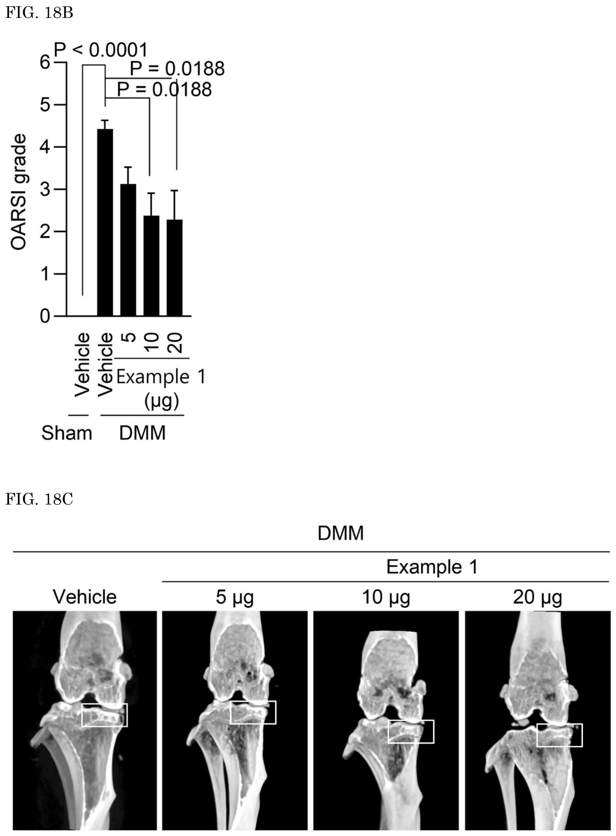

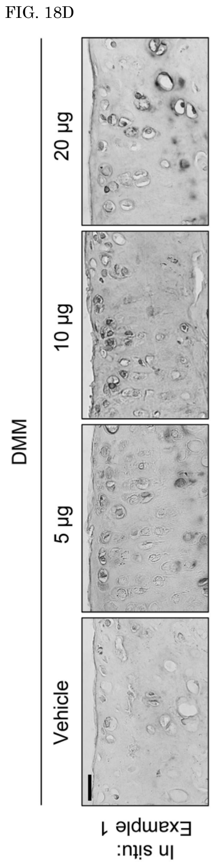

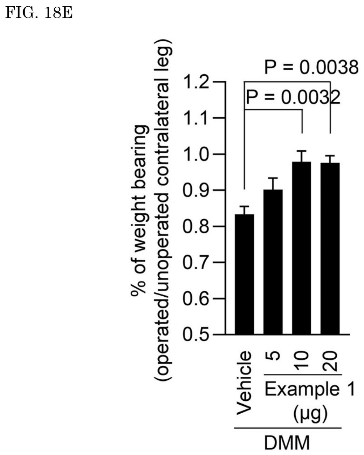

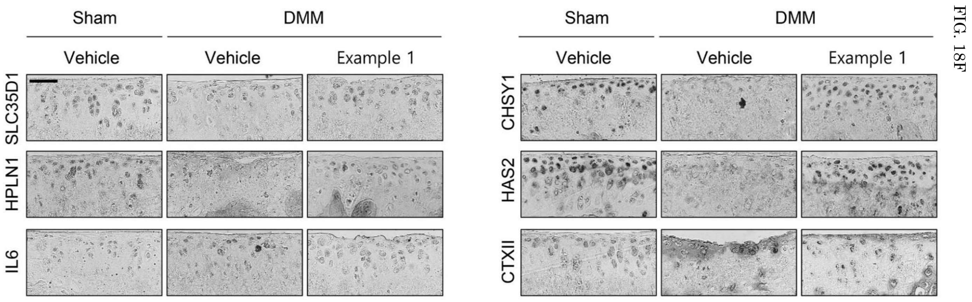

FIGS. 18A to 18F show the therapeutic effects of single intra-articular administration of Example 1 according to an embodiment of the present invention in a destabilization of the medial meniscus (DMM) surgery-induced OA mouse model. FIG. 18A shows histological images of Safranin O staining after administration of Example 1 and a vehicle control. FIG. 18B shows a graph quantifying cartilage damage intensity and numerically representing pathological progression stages based on the images in FIG. 18A. FIG. 18C shows images of microstructural changes in cartilage tissue through micro-computed tomography (µCT). FIG. 18D shows images confirming the distribution of Example 1 within articular cartilage through *in situ* hybridization. FIG. 18E shows a graph illustrating the ameliorative effect of Example 1 on weight-bearing imbalance in the DMM surgery-induced mouse OA model. FIG. 18F shows images of immunohistochemistry analysis results representing changes in expression of proteins related to cartilage matrix synthesis and degradation.

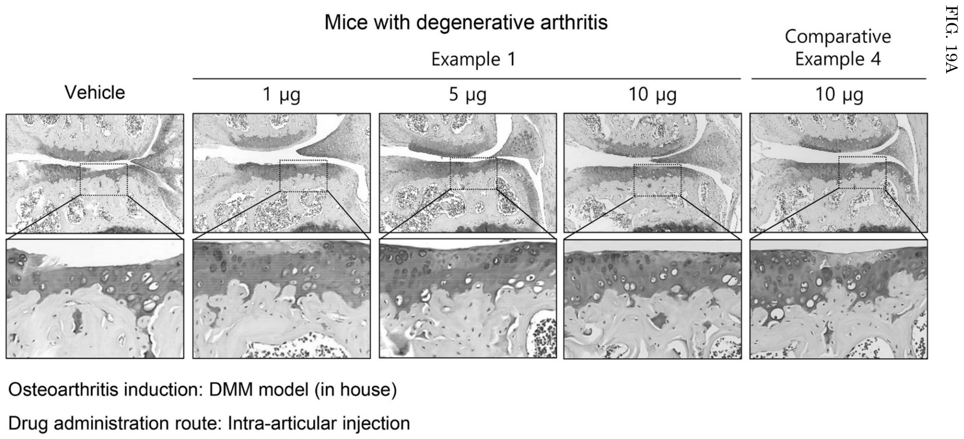

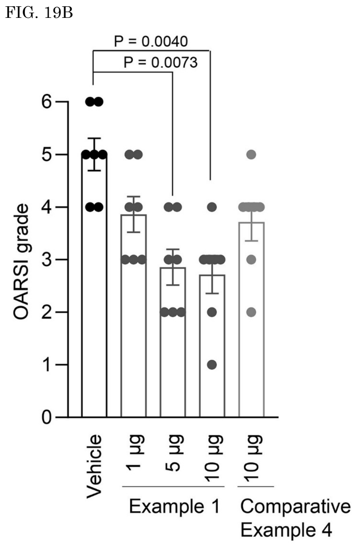

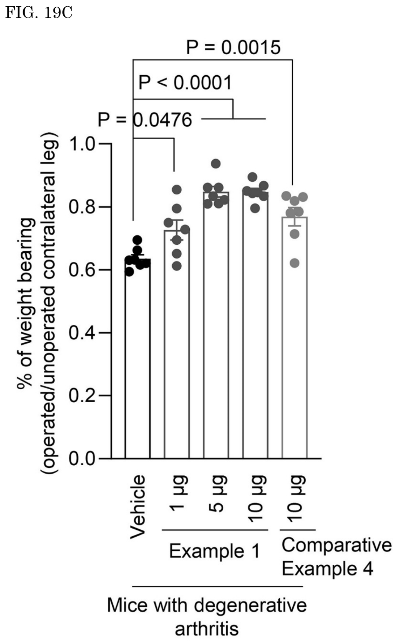

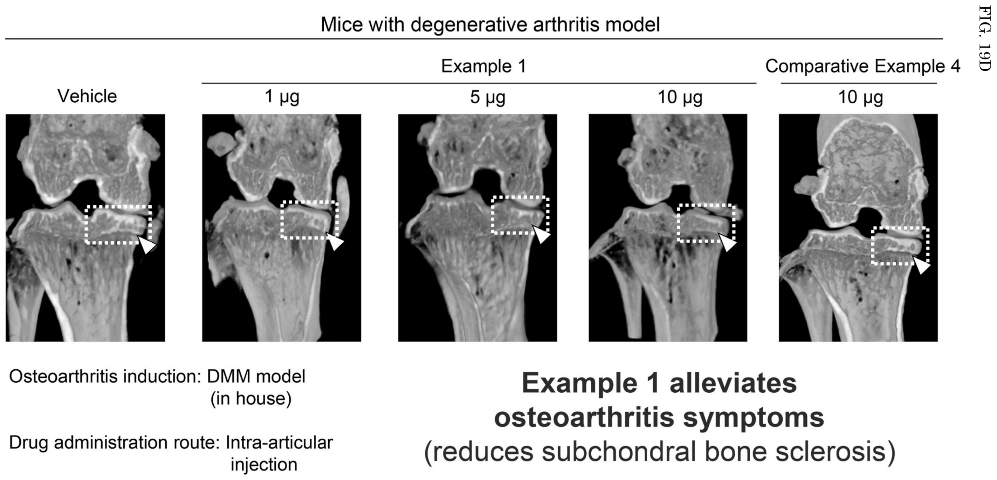

FIGS. 19A to 19D show the therapeutic effects of twice-repeated intra-articular administration of Example 1 as an embodiment of the present invention the DMM surgery-induced mouse OA model. FIGS. 19A and 19B show images of histological analysis of cartilage in an Example 1 treatment group and a vehicle control group through Safranin O staining and a graph quantifying the pathological progression stages. FIG. 19C shows a graph illustrating the ameliorating effect of Example 1 on weight-bearing imbalance. FIG. 19D shows images illustrating microstructural changes in cartilage tissue structure through µCT.

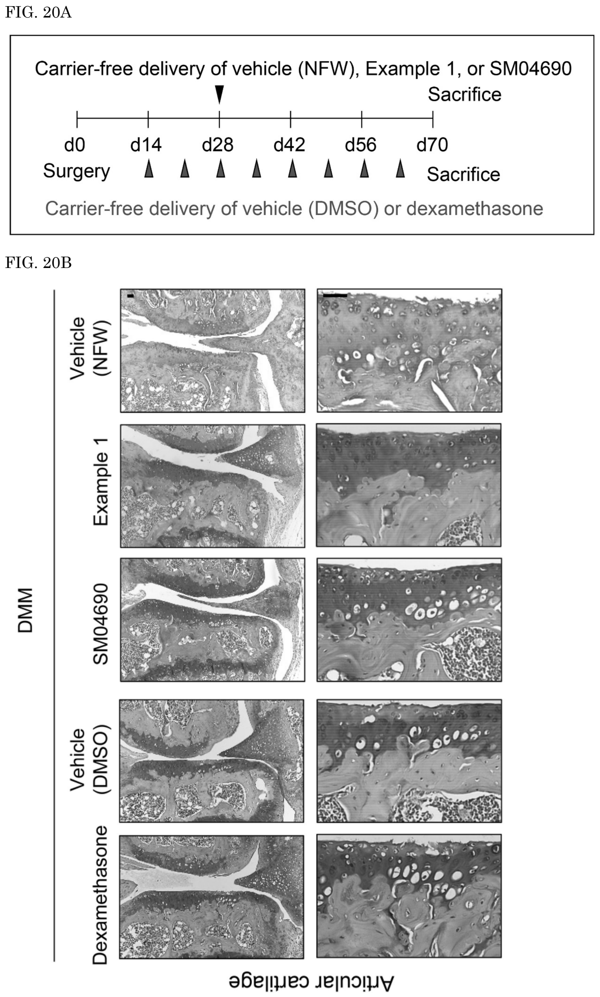

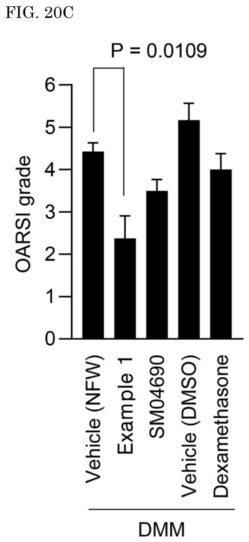

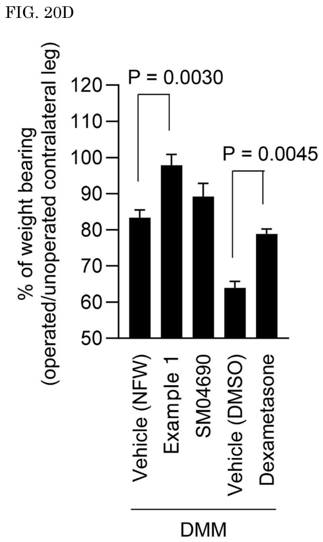

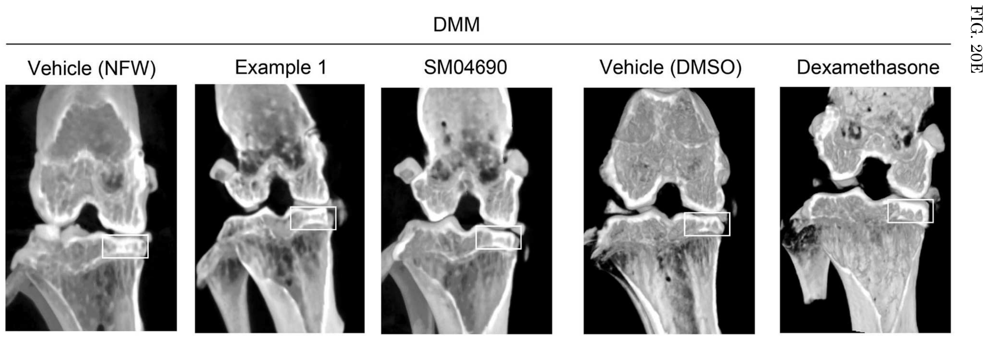

FIGS. 20A to 20E show the experimental results for comparing the therapeutic effects of Example 1, SM04690, and dexamethasone as an embodiment of the present invention in a mouse osteoarthritis model induced by DMM surgery. FIG. 20A shows a schematic diagram illustrating the treatment duration and treatment conditions for each drug. FIGS. 20B and 20C show Safranin O staining histological images of each drug treatment group and a graph quantifying the pathological progression stages. FIG. 20D shows a graph illustrating the results of weight-bearing imbalance analysis in each drug treatment group through behavioral experiments. FIG. 20E shows images illustrating microstructural changes in cartilage tissue structure through µCT.

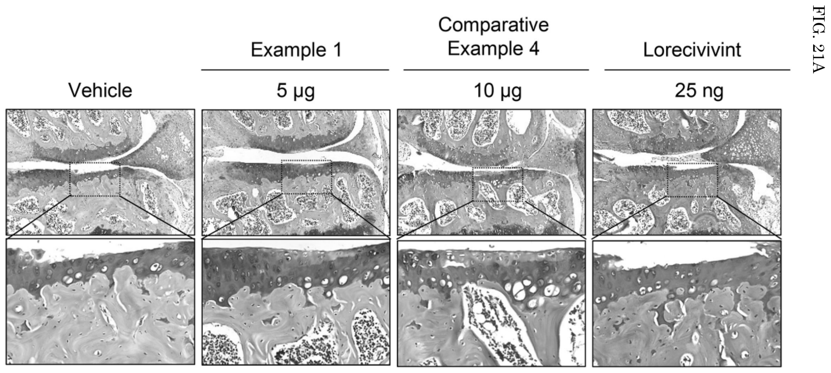

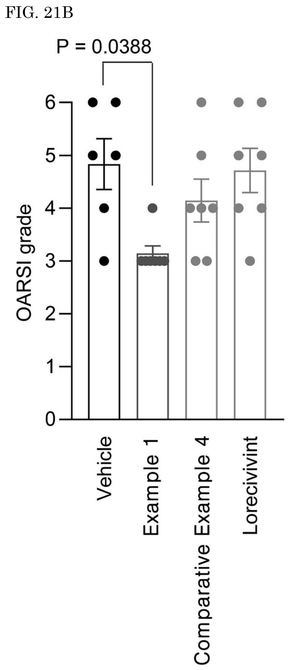

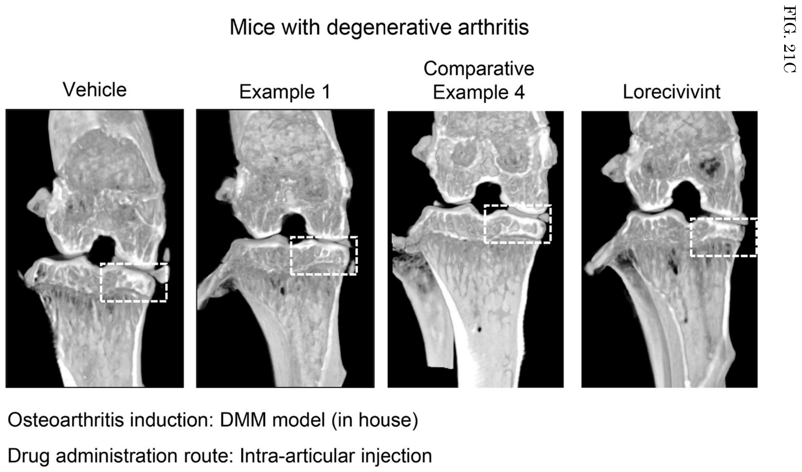

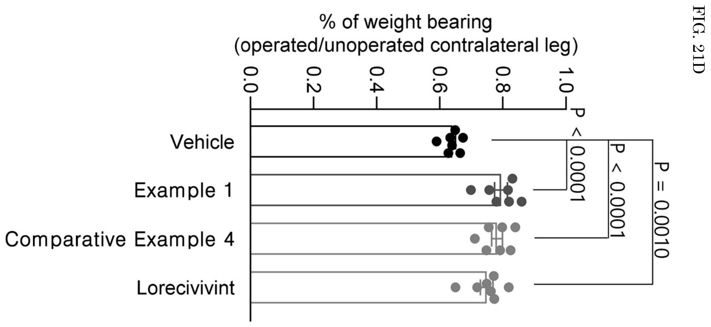

FIGS. 21A to 21D show the results of evaluating the therapeutic effect of Example 1 as an embodiment of the present invention in the late stage of OA. FIGS. 21A and 21B show Safranin O staining histological images of each drug treatment group and a graph quantifying the pathological progression stages. FIG. 21C shows images illustrating microstructural changes in cartilage tissue structure in each drug treatment group through µCT. FIG. 21D shows a graph illustrating the results of weight-bearing imbalance analysis in each drug treatment group through behavioral experiments.

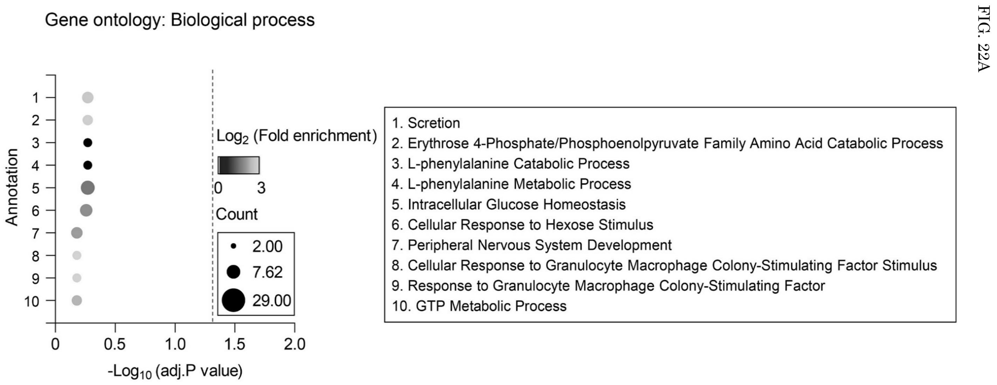

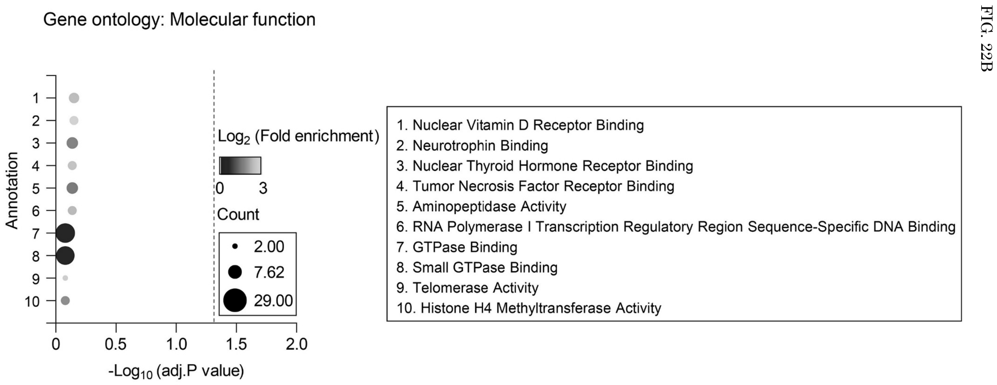

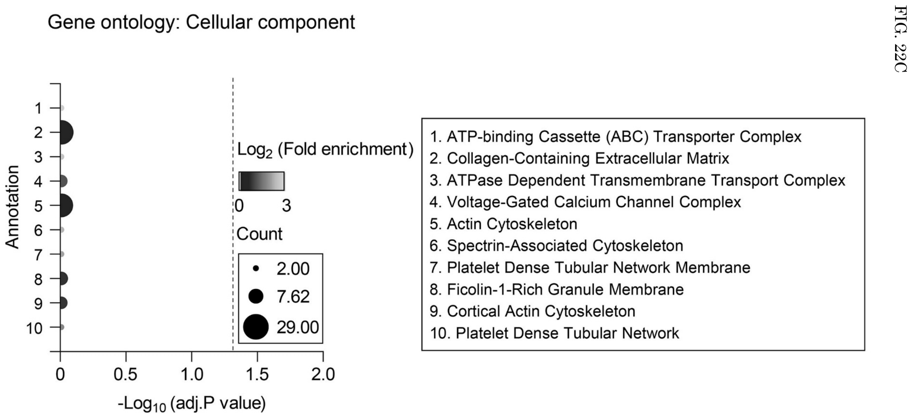

FIGS. 22A to 22C show analysis results for evaluating the off-target effects of Example 1 as an embodiment of the present invention. FIGS. 22A to 22C show bubble plot graphs illustrating the gene ontology (GO) term analysis results using transcriptome data obtained after treating C28/I2 cell lines overexpressing miR-204 with Example 1. The size of the dots represents the number of genes included in the corresponding annotation, and the color represents the fold enrichment.

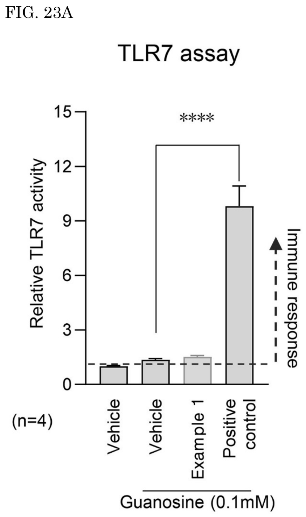

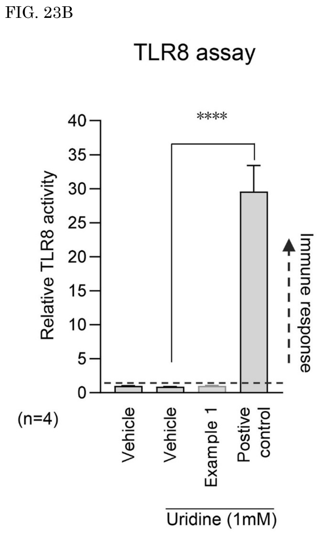

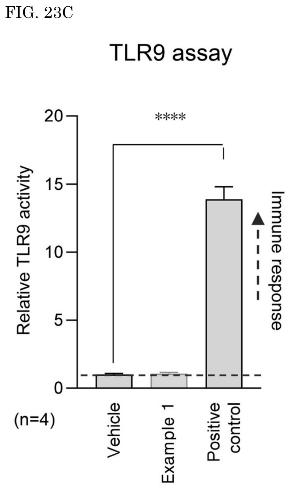

FIGS. 23A to 23C show graphs illustrating the immunogenicity evaluation results of Example 1 by conducting Toll-like receptor (TLR) activity analysis using HEK-Blue TLR7, TLR8, and TLR9 cell lines.

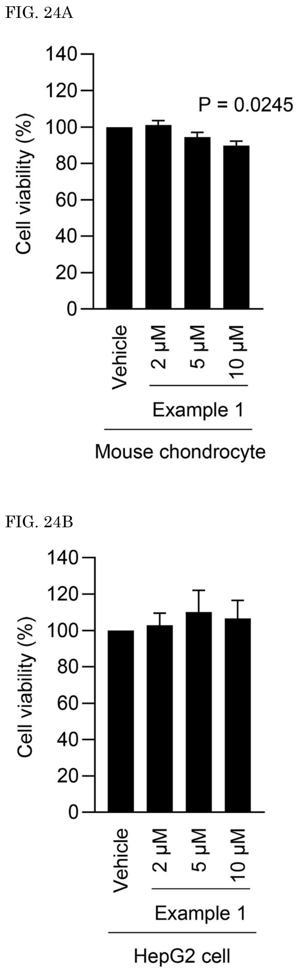

FIGS. 24A to 24C show the cytotoxicity evaluation results of Example 1 as an embodiment of the present invention. FIGS. 24A to 24C show graphs illustrating cell viability in mouse primary cultured chondrocytes, hepatocyte cell lines, and renal cell lines, respectively, when treated with Example 1 at various concentrations.

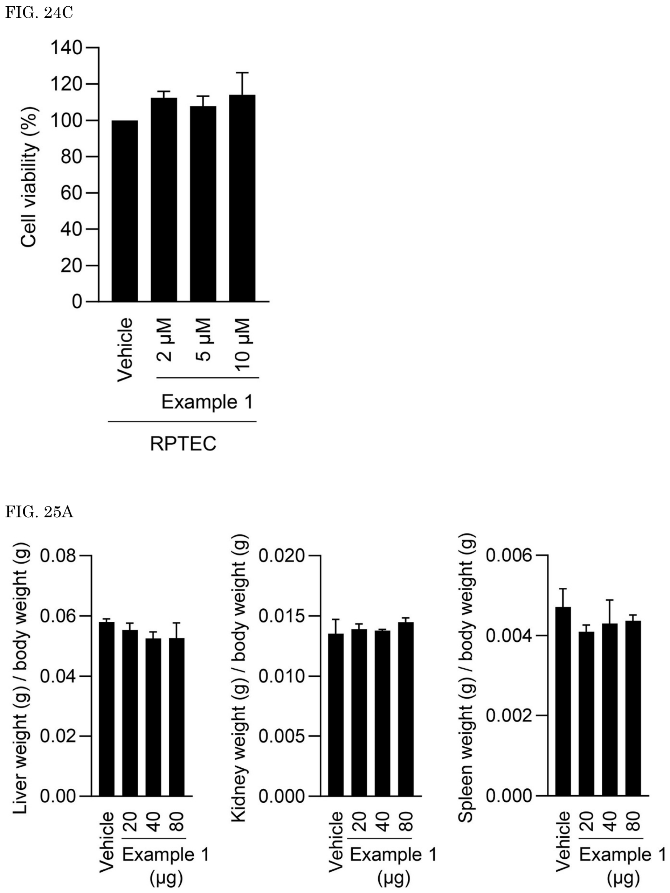

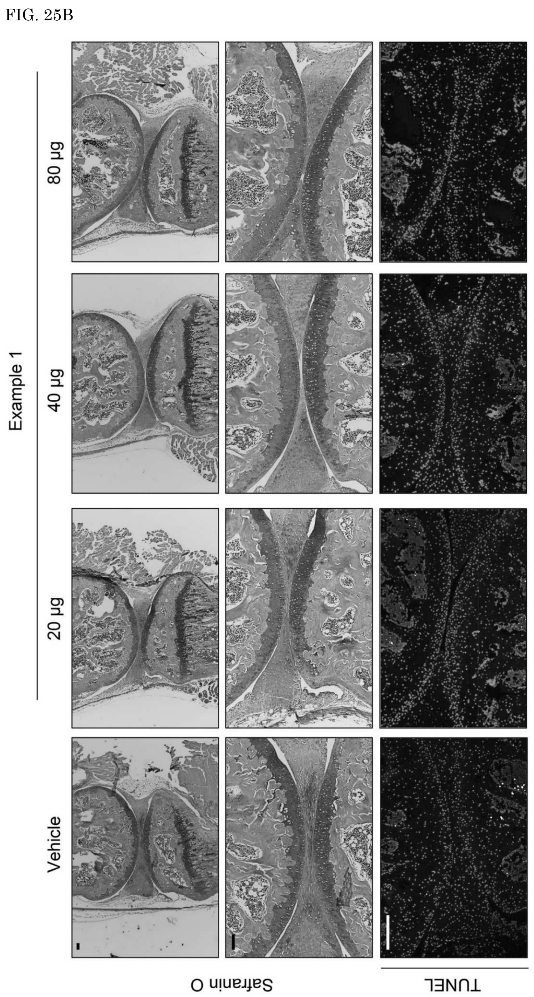

FIGS. 25A to 25B show the toxicity evaluation results after single intra-articular administration of Example 1 to mice as an embodiment of the present invention. FIG. 25A shows a graph illustrating the organ-to-body weight ratio of major organs (liver, kidney, spleen) measured 14 days after administration. The upper and middle portions of FIG. 25B show images illustrating Safranin O staining results of knee joint tissue, and the lower portion shows images illustrating Terminal deoxynucleotidyl transferase-mediated dUTP nick-end labeling (TUNEL) staining analysis results of the same tissue.

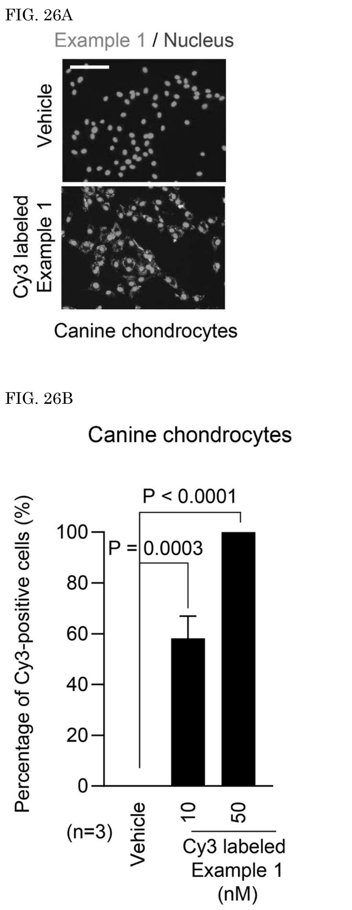

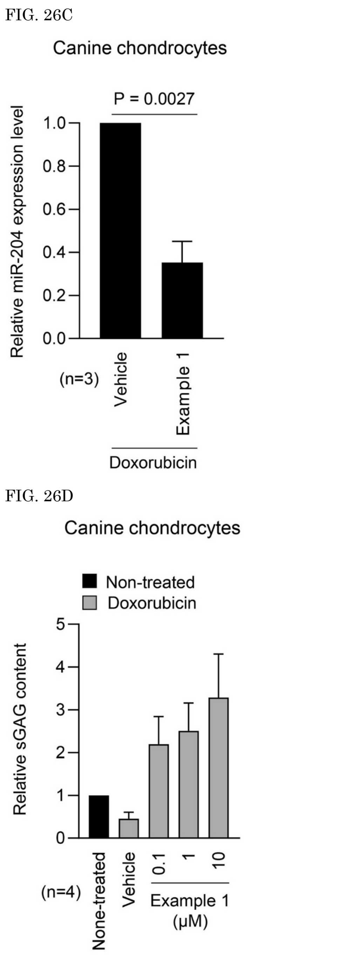

FIGS. 26A to 26D show the results of confirming intracellular delivery efficiency and functional efficacy of Example 1 as an embodiment of the present invention in canine chondrocytes. FIG. 26A shows fluorescence microscopic images of canine chondrocytes treated with Cy3 fluorescently-labeled Example 1. FIG. 26B shows a graph illustrating the results of quantifying the proportion of fluorescence-positive cells through flow cytometry after treatment with Cy3 fluorescently-labeled Example 1. FIG. 26C shows a graph illustrating the results of measuring miR-204 expression levels by qRT-PCR when Example 1 was treated under cellular senescence conditions. FIG. 26D shows a graph illustrating the results of quantitative analysis of sGAG content under cellular senescent conditions.
Hereinafter, the present invention will be described in detail.

In one aspect of the present invention, the term "microRNA-204," "miR-204," or "microRNA-204" is a collective term encompassing any variants, isoforms, and ortholog of miR-204 that are naturally expressed by cells. Specifically, the term may refer to human miR-204, but is not limited thereto, and may include miR-204 from other animals.

In one aspect of the present invention, the term "tail region of miR-204" may be a nucleic acid region from nucleotide 17 onward from the 5' end of microRNA-204 (miR-204).

In one aspect of the present invention, the term "supplementary region of miR-204" may be a nucleic acid region from nucleotides 13 to 16 from the 5' end of microRNA-204 (miR-204).

In one aspect of the present invention, the term "central region of miR-204" may be a nucleic acid region from nucleotides 9 to 12 from the 5' end of microRNA-204 (miR-204).

In one aspect of the present invention, the term "seed region of miR-204" may be a nucleic acid region from nucleotides 2 to 8 from the 5' end of microRNA-204 (miR-204).

In one aspect of the present invention, the term "oligonucleotide" may mean a short nucleic acid polymer that exhibits function by complementarily binding to a target nucleic acid, specifically the target and microRNA, and the polymer may include natural or modified nucleotides, for example, sugar modifications such as 2'-O-methoxyethyl (MOE), 2'-O-methyl (OME), 2'-fluoro (2'-F), a locked nucleic acid (LNA), a constrained ethyl (cEt), and the like, and a phosphorothioate (PS) bond. In one aspect, the term "oligonucleotide" may be an antisense oligonucleotide.

In one aspect of the present invention, the term "nucleotide including a constrained sugar modification" may mean that the sugar included in the nucleotide has undergone a modification that conformationally constrains the stereochemical configuration of the sugar. In one aspect, the nucleotide including a constrained sugar modification may be a nucleotide including a bicyclic sugar (e.g., LNA, 2',4'-ethylene-bridged nucleic acid (ENA), cEt, bridged nucleic acid (BNA), acyclic amino-bridged BNA (BNANC,) etc.).

In one aspect of the present invention, the term "a nucleotide including a non-constrained 2'-substituted sugar modification" may mean that the sugar included in the nucleotide has undergone modification based on a 2'-position substitution such that the stereochemical configuration of the sugar is non-constrained or non-fixed. In one aspect, the non-constrained 2'-substituted sugar modification may include a non-bicyclic sugar modification (e.g., 2'-deoxy, 2'-O-methyl (2'-O-Me), 2'-O-methoxyethyl (2'-O-MOE), 2'-O-alkyl, 2'-fluoro (2'-F), 2'-amino (2'-NH₂), 2'-O-allyl, 2'-O-benzyl, ribose, arabinose (ANA), etc.).

In one aspect of the present invention, the term "osteoarthritis (OA)" is a group of diseases encompassing degenerative damage or dysfunction of various joint tissues, such as joint cartilage, subchondral bone, ligaments, and synovium, and may include primary or secondary OA, OA of all sites including the knees, hip, hands, and spine.

### Oligonucleotide specifically hybridizing with miR-204

In one aspect, the present invention provides an oligonucleotide specifically hybridizing with miR-204, wherein the oligonucleotide includes 20 to 22 linked nucleotides and consists of a first region, a second region, and a third region in order from a 5' end to a 3' end, wherein the first region includes three consecutively connected nucleotides, each complementary to a tail region of miR-204 and including a constrained sugar modification, the second region includes three connected nucleotides connected, the nucleotides included in the second region each independently being nucleotides including a constrained sugar modification or a non-constrained 2'-substituted sugar modification, and the third region includes a plurality of connected nucleotides, the nucleotides included in the third region each independently being nucleotides including a non-constrained 2'-substituted sugar modification, and wherein the oligonucleotide includes 30% or less of nucleotides including a constrained sugar modification based on a total number of nucleotides.

The oligonucleotide according to one aspect of the present invention may specifically hybridize with miR-204.

The oligonucleotide according to one aspect of the present invention may include 20 to 22 linked nucleotides. Specifically, the oligonucleotide may include 20, 21, or 22 linked nucleotides.

The oligonucleotide according to one aspect of the present invention may include a first region, a second region, and a third region in order from a 5' end to a 3' end. Specifically, the first region may be a region including nucleotides 1 to 3 of the oligonucleotide in order from a 5' end to a 3' end. In addition, the second region may be a region including nucleotides 4 to 6 of the oligonucleotide in order from a 5' end to a 3' end. In addition, the third region may be a region including nucleotides 7 to last (e.g., 20th, 21st, or 22nd) of the oligonucleotide in order from a 5' end to a 3' end. More specifically, the first region may be a region consisting of nucleotides 1 to 3 of the oligonucleotide in order from a 5' end to a 3' end. In addition, the second region may be a region consisting of nucleotides 4 to 6 of the oligonucleotide in order from a 5' end to a 3' end. In addition, the third region may be a region consisting of nucleotides 7 to last (e.g., 20th, 21st, or 22nd) of the oligonucleotide in order from a 5' end to a 3' end.

The oligonucleotide according to one aspect of the present invention may be designed to enhance binding affinity, *in vivo* stability and functional inhibitory effects by selectively introducing an LNA, 5-methyl-deoxycytosine (iMe-dC), a PS bond, a PO bond, and the like.

An LNA base has a bicyclic structure with a constrained sugar conformation, which may contribute to enhancing ligand binding strength and structural rigidity of the oligonucleotide. In addition, iMe-dC is a modified base with increased resistance to oxidation and deamination compared to wild-type cytosine, and therefore, it is the most standardized synthetic base modification for stably and reproducibly mimicking actual biological phenomena, and may contribute to increasing the structural stability and functional persistence of an entire antisense oligonucleotide (ASO). In addition, a PS bond may increase nuclease resistance and increase protein binding affinity, thereby contributing to prolonging the *in vivo* half-life.

Meanwhile, the nucleotides included in, or constituting the oligonucleotide according to one aspect of the present invention may be linked by a PS bond and/or a PO bond.

According to Experimental Example 1, in the ASO treatment group of Example 1, which is one embodiment of the present invention, the expression of miR-204 decreased in a concentration-dependent manner, and quantitative analysis results showed that the maximum degradation efficiency was about 99.6%, and the DC₅₀ that degrades 50% of miR-204 expression was calculated to be about 0.0055 µM, exhibiting high degradation ability (FIG. 1). Furthermore, excellent miR-204 degradation ability was confirmed in the ASO treatment group of Example 2, which is one embodiment of the present invention, confirming that the oligonucleotide according to one aspect of the present invention can effectively degrade the target miR-204 regardless of the internucleotide linkage method or backbone components of the oligonucleotide (FIG. 14).

A microRNA is generally composed of 22 to 24 nucleotides, and nucleotides 2 to 8 from a 5' end constitute a seed region, nucleotides 9 to 12 constitute a central region, nucleotides 13 to 16 constitute a supplementary region, and the remainder constitutes a tail region. The first region of the ASO of Example 1, which is one embodiment of the present invention, has a sequence completely complementary to the tail region.

According to Experimental Example 2, the Cy5.5-labeled ASO of Example 1 was confirmed by strong fluorescent signals in the cytoplasm of chondrocytes, and flow cytometry analysis showed that intracellular fluorescence intensity increased in a concentration-dependent manner in the Cy3-labeled Example 1 treatment group. Therefore, the results show that the structural design of the ASO according to one embodiment of the present invention can contribute to effective delivery and uptake even in cells with high penetration difficulty, such as cartilage tissue (FIG. 2).

The oligonucleotide according to one aspect of the present invention can be efficiently delivered into chondrocytes or cartilage tissue. Specifically, the oligonucleotide according to one aspect of the present invention can effectively induce cellular uptake into chondrocytes or retention within cartilage tissue.

According to Experimental Example 3, the ASO of Example 1 according to one embodiment of the present invention was well delivered into cells in human-derived cartilage tissue (FIG. 3), which suggests that the ASO according to one embodiment of the present invention has physicochemical properties that enable stable accumulation in human chondrocytes.

According to Experimental Example 4, it was confirmed that variants (Examples 3-1 to 3-3) containing single or a plurality of mismatches in the central region or supplementary region of miR-204 have no significant decrease in binding efficiency compared to a fully complementary ASO and maintain excellent degradation ability (FIG. 4). These results suggests that the sequence design of the ASO according to one embodiment of the present invention is very advantageous for securing high degradation efficiency, binding stability, and biological functionality.

The term "mismatch" as used herein means a state where a single base is non-complementarily substituted when compared to a complementary sequence of the target microRNA. In other words, the Watson-Crick complementary base pair expected at a specific position in the oligonucleotide is not formed, and the position may take the form of G·U wobble pairing or non-canonical pairing, but is not limited thereto.

The oligonucleotide according to one aspect of the present invention may include one or more mismatch nucleotides with the miR-204. Specifically, the mismatch may be located in a region complementary to the central region or supplementary region of miR-204. Furthermore, specifically, the mismatch nucleotides may be present in the oligonucleotide in numbers of 1 or more, 2 or more, 3 or more, 4 or more, 5 or more, 6 or more, 7 or more, 8 or more, 9 or more, or 10 or more, and may be present in numbers of 10 or less, 9 or less, 8 or less, 7 or less, 6 or less, 5 or less, 4 or less, 3 or less, or 2 or less.

The second region or third region of the oligonucleotide according to one aspect of the present invention may include one or more mismatch nucleotides with the miR-204. Specifically, the oligonucleotide may include mismatch nucleotides at one or more selected positions among nucleotides 1 to 22. More specifically, the oligonucleotide may include mismatch nucleotides at one or more selected positions among nucleotides 7 to 22, 7 to 20, 8 to 18, 8 to 16, or 8 to 14. Even more specifically, the oligonucleotide may include mismatch nucleotides at one or more selected positions among nucleotide 7, 8, 9, 10, 11, 12, 13, 14, 15, 16, 17, 18, 19, 20, 21, or 22, and preferably, may include mismatch nucleotides at one or more selected positions among nucleotide 8, 9, 10, 11, 12, 13, or 14.

Furthermore, the mismatch nucleotides may be present in a complementary sequence to any one or more positions among nucleotides 1 to 22 from the 5' end of the miR-204. Specifically, the oligonucleotide may include mismatch nucleotides at one or more selected positions among nucleotides 1 to 22, 1 to 16, 3 to 16, 6 to 16, or 9 to 15 of miR-204. More specifically, the oligonucleotide may include mismatch nucleotides at one or more selected positions among nucleotide 1, 2, 3, 4, 5, 6, 7, 8, 9, 10, 11, 12, 13, 14, 15, or 16 of miR-204, and even more specifically, may include mismatch nucleotides at one or more selected positions among nucleotide 9, 10, 11, 12, 13, 14, or 15.

According to Experimental Example 5, it was confirmed that ASOs (Examples 4-1 and 4-2) with one or two bases truncated from the 3'-end of ASO compared to a fully complementary ASO significantly degrade miR-204, and binding affinity and degradation ability for miR-204 were maintained even at shortened lengths (FIG. 5).

These results suggest that some truncation at the 3' terminus in miR-204-targeted ASO design does not impair functional efficacy and can maintain miR-204 degradation ability.

The nucleotide including a constrained sugar modification according to one aspect of the present invention may be at least one selected from the group consisting of LNA, ENA, cEt nucleotide, BNA, and BNANC. Specifically, the nucleotide including the constrained sugar modification may be at least one of LNA and ENA, and more specifically, it may be LNA.

The non-constrained 2'-substituted sugar modification according to one aspect of the present invention may be at least one selected from the group consisting of non-constrained 2'-substituted sugar modifications of 2'-deoxy, 2'-O-methyl (2'-O-Me), 2'-O-methoxyethyl (2'-O-MOE), 2'-O-alkyl, 2'-fluoro (2'-F), 2'-amino (2'-NH₂), 2'-O-allyl, 2'-O-benzyl, ribose, and ANA. More specifically, the non-constrained 2'-substituted sugar modification may be at least one selected from the group consisting of 2'-deoxy, 2'-O-methyl, and 2'-O-methoxyethyl.

The oligonucleotide according to one aspect of the present invention may include a first region complementary to the tail region of miR-204, and the first region may be three consecutively linked nucleotides including a constrained sugar modification. Specifically, the first region may include nucleotides 1 to 3 from the 5' end to the 3' end of the oligonucleotide, and the three nucleotides may be three consecutively linked nucleotides including a constrained sugar modification. More specifically, the first region may consist of nucleotides 1 to 3 from the 5' end to the 3' end of the oligonucleotide, and the three nucleotides may be three consecutively linked nucleotides each consisting of a constrained sugar modification.

The oligonucleotide according to one aspect of the present invention may include a second region complementary to the tail region of miR-204, and the nucleotide included in the second region may each independently be a nucleotide including either a constrained sugar modification or a non-constrained 2'-substituted sugar modification. Specifically, the second region may be positioned after the first region from the 5' end to the 3' end of the oligonucleotide. In addition, the second region may include nucleotides 4 to 6 from the 5' end to the 3' end of the oligonucleotide, and the three nucleotides may be three consecutively linked nucleotides including a constrained sugar modification or a non-constrained 2'-substituted sugar modification. More specifically, the second region may consist of nucleotides 4 to 6 from the 5' end to the 3' end of the oligonucleotide, and the three nucleotides may be three consecutively linked nucleotides consisting of a constrained sugar modifications or a non-constrained 2'-substituted sugar modifications.

According to one embodiment of the present invention, the oligonucleotide according to one aspect of the present invention has stable and efficient degradation efficacy for miR-204 when the oligonucleotide includes a constrained sugar modification at positions 1 to 3 (first region) from the 5' end to the 3' end (Examples 5-1 to 5-7). In addition, the oligonucleotide exhibits the same miR-204 degradation efficacy even when a non-constrained 2'-substituted sugar modification and a constrained sugar modification are mixed at the adjacent positions 4 to 6 (second region). However, oligonucleotides including only one or two constrained sugar modifications in the first region (Comparative Examples 1-1 and 1-2) showed miR-204 expression levels similar to the control group at a concentration of 10 µM, thus exhibiting almost no miR-204 degradation efficacy (see Experimental Example 6-1, and FIGS. 6 and 7).

In addition, according to one embodiment of the present invention, the overall structural stability and miR-204 degradation efficacy of the oligonucleotide according to one aspect of the present invention were maintained even when the oligonucleotide included MOE or OME as a non-constrained 2'-substituted sugar modification at one or more of positions 4 to 6 (second region) from the 5' end to the 3' end (see Examples 6-1 to 6-14, Experimental Example 8, and FIGS. 12 and 13).

The oligonucleotide according to one aspect of the present invention may include a third region complementary to the supplementary region, central region, and seed region of miR-204, and the nucleotide included in the third region may each independently be a nucleotide including a non-constrained 2'-substituted sugar modification. Specifically, the third region may be positioned after the second region from the 5' end to the 3' end of the oligonucleotide. In addition, the third region may include nucleotides 7 to last from the 5' end to the 3' end of the oligonucleotide, and the plurality of nucleotides may be consecutively linked nucleotides including a non-constrained 2'-substituted sugar modification. More specifically, the third region may consist of nucleotides 7 to last from the 5' end to the 3' end of the oligonucleotide, and the plurality of nucleotides may be consecutively linked nucleotides consisting of a non-constrained 2'-substituted sugar modification. Even more specifically, all nucleotides constituting the third region may include 2'-deoxy.

The oligonucleotide according to one aspect of the present invention may include a region complementary to the seed region of miR-204, and the region complementary to the seed region of miR-204 may be a part of the third region of the oligonucleotide according to one aspect of the present invention, specifically a part adjacent to the 3' end. The sugars of nucleotides included in the region complementary to said seed region may include a non-constrained 2'-substituted sugar modification.

In the oligonucleotide according to one aspect of the present invention, the sugar of one or more nucleotides at positions 1 to 16 from the 3' end of the oligonucleotide may be 2'-deoxy. Specifically, in the oligonucleotide, the sugar of 1 or more, 2 or more, 3 or more, 4 or more, 5 or more, 6 or more, 7 or more, 8 or more, 9 or more, 10 or more, 11 or more, 12 or more, 13 or more, 14 or more, or 15 or more nucleotides from the 3' end of the oligonucleotide may be 2'-deoxy, and the sugar of 16 or fewer, 15 or fewer, 14 or fewer, 13 or fewer, 12 or fewer, 11 or fewer, 10 or fewer, 9 or fewer, 8 or fewer, 7 or fewer, 6 or fewer, 5 or fewer, 4 or fewer, 3 or fewer, or 2 or fewer nucleotides may be 2'-deoxy. More specifically, in the oligonucleotide, the sugar of nucleotides at positions 1 and 2, positions 1 to 3, positions 1 to 4, positions 1 to 5, positions 1 to 6, positions 1 to 7, positions 1 to 8, positions 1 to 9, positions 1 to 10, positions 1 to 11, positions 1 to 12, positions 1 to 13, positions 1 to 14, positions 1 to 15, or positions 1 to 16 from the 3' end of the oligonucleotide may be 2'-deoxy.

According to one embodiment of the present invention, when the oligonucleotide according to one aspect of the present invention did not include a constrained sugar modification in the first region, but included a constrained sugar modification only in the region proximal to the 3' end (third region) (Comparative Examples 2-1 to 2-4) or randomly included constrained sugar modification in the second and third regions (Comparative Examples 3 and 4), the binding affinity of the ASO and RNase H-mediated degradation efficiency were reduced so that the overall miR-204 degradation effect was decreased (see Experimental Examples 6-2 and 6-3, and FIGS. 8 to 10). Therefore, it was found that in the oligonucleotide according to one aspect of the present invention, the position of a constrained sugar modification has a critical impact on miR-204 degradation activity, and the best miR-204 degradation efficacy was exhibited when constrained sugar modifications were included near to the 5' end, that is, in the first region.

The oligonucleotide according to one aspect of the present invention may include 30% or less of nucleotides including a constrained sugar modification based on a total number of nucleotides. Specifically, the oligonucleotide may include nucleotides including a constrained sugar modification in an amount of 30% or less, 29.5% or less, 29% or less, 28.5% or less, 28% or less, 27.5% or less, 27.3% or less, 27% or less, 26% or less, 25% or less, 24% or less, 23% or less, 22.7% or less, 22% or less, 21% or less, 20% or less, 19% or less, 18.2% or less, 18% or less, 17% or less, 16% or less, 15% or less, 14% or less, 13.6% or less, 13% or less, 12% or less, 11% or less, or 10% or less, based on a total number of nucleotides. More specifically, the oligonucleotide may include 13% to 30% of nucleotides including a constrained sugar modification based on a total number of nucleotides.

Alternatively, the oligonucleotide according to one aspect of the present invention may include 3 to 14 nucleotides including a constrained sugar modification. Specifically, the oligonucleotide may include 3 or more, 4 or more, 5 or more, 6 or more, 7 or more, 8 or more, 9 or more, 10 or more, 11 or more, 12 or more, or 13 or more nucleotides including a constrained sugar modification, and may include 14 or fewer, 13 or fewer, 12 or fewer, 11 or fewer, 10 or fewer, 9 or fewer, 8 or fewer, 7 or fewer, 6 or fewer, 5 or fewer, or 4 or fewer nucleotides including a constrained sugar modification. More specifically, the oligonucleotide may include 3 to 6 nucleotides including a constrained sugar modification. Since the oligonucleotide according to one aspect of the present invention exhibits excellent miR-204 degradation efficacy when all three nucleotides constituting the first region include a constrained sugar modification, the nucleotides including a constrained sugar modification may be 13% or more or 3 or more.

According to one embodiment of the present invention, while the oligonucleotide according to one aspect of the present invention enhances miR-204 degradation efficacy as it includes more nucleotides including a constrained nucleic acid modification, it may simultaneously cause physical limitations and formulation limitations as a drug due to increased viscosity and decreased solubility of the formulation. Therefore, when considering both efficacy and formulation stability, a non-excessive level of nucleotides including a constrained nucleic acid modification (e.g., 13% to 30% or 3 to 6 nucleotides) was determined to be the optimal structure that maintains a balance between miR-204 degradation efficacy and formulation suitability (see Experimental Example 7 and FIG. 11B).

Each nucleotide included in the oligonucleotide according to one aspect of the present invention may be linked by a phosphorothioate bond or a phosphodiester bond. The internucleotide linkages included in the oligonucleotide according to one aspect of the present invention may each independently be a phosphorothioate bond or a phosphodiester bond. Specifically, in the oligonucleotide according to one aspect of the present invention, the internucleotide phosphate linkages may each independently be a phosphorothioate bond and/or phosphodiester bond. For example, some internucleotide linkages may be phosphorothioate bonds and others may be phosphodiester bonds, and the number and positions of these linkages are not particularly limited. As another example, all internucleotide linkages may be either phosphorothioate bonds or phosphodiester bonds.

The oligonucleotide according to one aspect of the present invention can effectively degrade miR-204 in chondrocytes. The oligonucleotide according to one aspect of the present invention may exhibit an effect of preventing or treating OA. Specifically, the oligonucleotide according to one aspect of the present invention may exhibit an effect of preventing or treating OA by effectively degrading miR-204 in chondrocytes.

In one embodiment, the OA may be at least one selected from the group consisting of degenerative (primary) OA, post-traumatic OA, post-surgical OA, inflammatory OA, and secondary OA.

The oligonucleotide according to one aspect of the present invention is not limited to extracellular matrix (ECM) remodeling-related genes (e.g., ECM proteoglycan synthesis factors) and/or pro-inflammatory genes (e.g., IL6, PTGS2, NOS2, IL family, matrix degradation products) and/or cartilage matrix-degrading enzyme-related genes (e.g., matrix metalloproteinases (MMP) family (such as MMP3), a disintegrin and metalloprotease with thrombospondin motifs (ADAMTS) family).

The oligonucleotide according to one aspect of the present invention may exhibit an effect of preventing or treating OA even upon repeated administration. Specifically, the oligonucleotide according to one aspect of the present invention may exhibit stable and reproducible effects even upon repeated administration.

The oligonucleotide according to one aspect of the present invention can alleviate OA symptoms (e.g., cartilage damage, subchondral bone sclerosis, subchondral bone sclerosis) upon repeated administration. According to one embodiment of the present invention, when the oligonucleotide according to one aspect of the present invention is repeatedly administered twice, both cartilage damage and subchondral bone sclerosis can be significantly reduced (see Experimental Example 10 and FIGS. 19A, 19B, and 19D).

The oligonucleotide according to one aspect of the present invention can exhibit a pain alleviation effect even upon repeated administration. According to one embodiment of the present invention, the pain alleviation effect can be sustained when the oligonucleotide according to one aspect of the present invention is administered twice repeatedly (see Experimental Example 10 and FIG. 19C).

In one embodiment, repeated administration means administering the oligonucleotide according to one aspect of the present invention two or more times. Specifically, the repeated administration may mean administering the oligonucleotide in the same or different doses two or more times at regular intervals. For example, the repeated administration may be administering two, three, four, five or more times, but is not limited thereto.

The oligonucleotide according to one aspect of the present invention may exhibit reproducible efficacy upon both single administration and repeated administration.

The oligonucleotide according to one aspect of the present invention may have superior effect of preventing or treating OA compared to oligonucleotides that do not follow the sugar modification pattern of the oligonucleotide according to one aspect of the present invention. According to one embodiment of the present invention, the oligonucleotide according to one aspect of the present invention may exhibit significantly superior effects in terms of reducing cartilage damage, reducing subchondral bone sclerosis, and/or alleviating pain compared to other oligonucleotides that do not follow the sugar modification pattern of the oligonucleotide according to one aspect of the present invention under the same conditions (see Experimental Example 10 and FIGS. 19A to 19D).

The oligonucleotide according to one aspect of the present invention may exhibit superior preventive or therapeutic effects compared to existing OA therapeutics (e.g., commercially available OA therapeutics, OA therapeutics currently used in clinical practice or under development). Specifically, the oligonucleotide according to one aspect of the present invention may have superior cartilage protective effects, subchondral bone sclerosis amelioration effects, pain alleviation effects, and/or cartilage regeneration effects compared to OA therapeutics currently used in clinical practice or under development. For example, the existing OA therapeutic may be SM04690, or a steroidal anti-inflammatory analgesic (e.g., dexamethasone), but is not limited thereto.

According to one embodiment of the present invention, the oligonucleotide according to one aspect of the present invention may exhibit superior cartilage protective effects compared to SM04690 (see Experimental Example 10 and FIGS. 20A to 20E). According to one embodiment of the present invention, the oligonucleotide according to one aspect of the present invention may have superior cartilage regeneration capability compared to dexamethasone (see Experimental Example 10 and FIGS. 20A to 20E).

The oligonucleotide according to one aspect of the present invention can protect and/or restore cartilage structure. Specifically, the oligonucleotide according to one aspect of the present invention can ameliorate OA by inducing protection and/or restoration of the cartilage structure itself as well as regulation of inflammation.

The oligonucleotide according to one aspect of the present invention can exhibit preventive or therapeutic effects even in the late stage of OA. Specifically, the oligonucleotide according to one aspect of the present invention can restore cartilage even when administered in the late stage of advanced OA. The oligonucleotide according to one aspect of the present invention can exhibit superior cartilage recovery effects compared to a steric blocker and/or SM04690 in the late stage of OA. According to one embodiment of the present invention, the oligonucleotide according to one aspect of the present invention can exhibit statistically significant levels of cartilage recovery effects compared to a steric blocker (SEQ ID NO: 22) and SM04690 when administered six weeks after OA induction (see Experimental Example 10 and FIGS. 21A to 21C).

The oligonucleotide according to one aspect of the present invention can exhibit pain alleviation effects even in the late stage of OA. According to one embodiment of the present invention, the oligonucleotide according to one aspect of the present invention can exhibit statistically significant levels of pain alleviation effects when administered six weeks after OA induction (see Experimental Example 10 and FIG. 21D).

In one embodiment, the late stage of OA refers to a stage where OA has progressed for a certain period. Specifically, the late stage of OA may refer to a stage where pathological changes of OA have progressed such that cartilage damage, subchondral sclerosis, osteophyte development, and/or synovial inflammation are observed. For example, the late stage of OA may be a time point of 4 weeks, 5 weeks, 6 weeks, or more after OA induction in an animal model, but is not limited thereto. For example, the late stage of OA may be a time point of 4 weeks, 5 weeks, 6 weeks, or more after OA diagnosis, but is not limited thereto. In humans, the late stage of OA may be a time point of Kellgren-Lawrence (KL) grade 3, 4, or higher, but is not limited thereto. The late stage of OA refers to a state where pathological changes are more advanced and established compared to the early stage, and may include stages where treatment is more difficult.

The oligonucleotide according to one aspect of the present invention may have high sequence specificity for miR-204 and extremely low off-target effects.

The oligonucleotide according to one aspect of the present invention may have high specificity for miR-204. Specifically, the oligonucleotide according to one aspect of the present invention may not bind non-specifically to targets (e.g., human mRNA, microRNA, or pre-mRNA) other than miR-204 and/or its host gene transient receptor potential melastatin 3 (TRPM3). According to one embodiment of the present invention, National Center for Biotechnology Information-Basic Local Alignment Search Tool (NCBI-BLAST) analysis of the nucleotide sequence of the oligonucleotide according to one aspect of the present invention revealed no sequences expected to bind other than miR-204 and TRPM3 (see Experimental Example 11 and Table 14).

The oligonucleotide according to one aspect of the present invention may have a low off-target effect. Specifically, the oligonucleotide according to one aspect of the present invention may not have a significant effect on any other gene or biological pathway other than miR-204 degradation. According to one embodiment of the present invention, a gene ontology (GO) analysis using transcriptome data of cells treated with the oligonucleotide according to one aspect of the present invention did not provide significant ontology terms in any of the Biological Process, Molecular Function, and Cellular Component categories (see Experimental Example 11 and FIGS. 22A to 22C).

The oligonucleotide according to one aspect of the present invention may have low immunogenicity. Specifically, an oligonucleotide according to one aspect of the present invention may not induce an immune response through a Toll-like receptor (TLR). For example, the TLR may be TLR7, TLR8, and/or TLR9, but is not limited thereto.

The oligonucleotide according to one aspect of the present invention may not increase TLR activity. According to one embodiment of the present invention, no significant increase in the activity of TLR7, TLR8, and TLR9 was observed when treated with the oligonucleotide according to one aspect of the present invention (see Experimental Example 12 and FIGS. 23A to 23C).

The oligonucleotide according to one aspect of the present invention may have non-immunostimulatory properties with respect to innate immune receptor pathways. The oligonucleotide according to one aspect of the present invention may be an antisense oligonucleotide that does not induce immunogenicity.

The oligonucleotide according to one aspect of the present invention may exhibit no cytotoxicity or low cytotoxicity. Specifically, the oligonucleotide according to one aspect of the present invention may exhibit no cytotoxicity or low cytotoxicity in chondrocytes, hepatocytes, and/or renal cells. According to one embodiment of the present invention, the oligonucleotide according to one aspect of the present invention did not reduce cell viability to 80% or below in mouse cartilage-derived primary cultured cells, hepatocyte cell line (HepG2 cell line), and renal cell line (renal proximal tubule epithelial cells (RPTEC) cell line) (see Experimental Example 13 and FIGS. 24A to 24C).

The oligonucleotide according to one aspect of the present invention may exhibit no systemic toxicity. The oligonucleotide according to one aspect of the present invention may exhibit no systemic toxicity when administered intra-articularly. The oligonucleotide according to one aspect of the present invention may exhibit no toxicity to the liver, kidneys, and/or spleen. The oligonucleotide according to one aspect of the present invention may exhibit no toxicity to the liver, kidneys, and/or spleen when administered intra-articularly. According to one embodiment of the present invention, after a single intra-articular administration of the oligonucleotide according to one aspect of the present invention, the organ-to-body weight ratios of the liver, kidneys, and spleen did not show significant differences from the control group (see Experimental Example 13 and FIG. 25A).

The oligonucleotide according to one aspect of the present invention may exhibit no local toxicity. The oligonucleotide according to one aspect of the present invention may exhibit no local toxicity when administered intra-articularly. Specifically, the oligonucleotide according to one aspect of the present invention may induce no cartilage damage, proteoglycan loss, and/or chondrocyte apoptosis. Specifically, the oligonucleotide according to one aspect of the present invention may induce no cartilage damage, proteoglycan loss, and/or chondrocyte apoptosis when administered intra-articularly. According to one embodiment of the present invention, when the oligonucleotide according to one aspect of the present invention was administered intra-articularly, no cartilage damage or proteoglycan loss was observed within the knee joint tissue, the matrix components within the cartilage were maintained normally, and no chondrocyte apoptosis was induced (see Experimental Example 13 and FIG. 25B).

The oligonucleotide according to one aspect of the present invention can have excellent local safety and/or systemic safety. Specifically, the oligonucleotide according to one aspect of the present invention may be a safe ASO having no or low local toxicity and/or systemic toxicity at an administration site.

The oligonucleotide according to one aspect of the present invention can be efficiently delivered into chondrocytes. In addition, the oligonucleotide according to one aspect of the present invention can be efficiently delivered into mammalian-derived (e.g., canine) chondrocytes. Specifically, the oligonucleotide according to one aspect of the present invention can be efficiently delivered into mammalian-derived (e.g., canine) chondrocytes and can be stably accumulated within the chondrocytes. According to one embodiment of the present invention, the fluorescently-labeled oligonucleotide according to one aspect of the present invention was distinctly accumulated within canine chondrocytes, and fluorescent positive signals were observed in more than 50% of cells under conditions of treating the cells at 10 nM and 50 nM concentrations (see Experimental Example 14 and FIGS. 26A and 26B).

The oligonucleotide according to one aspect of the present invention may reduce miR-204 in canine chondrocytes. According to one embodiment of the present invention, the oligonucleotide according to one aspect of the present invention may statistically significantly reduce miR-204 in canine chondrocytes in which cellular senescence has been induced (see Experimental Example 14 and FIG. 26C).

The oligonucleotide according to one aspect of the present invention can restore cartilage matrix synthesis function in canine chondrocytes. Specifically, the oligonucleotide according to one aspect of the present invention can increase or restore the synthesis amount of sulfated glycosaminoglycan (sGAG) in canine chondrocytes. According to one embodiment of the present invention, the oligonucleotide according to one aspect of the present invention can statistically significantly increase the synthesis amount of sGAG reduced by senescence in canine chondrocytes in which cellular senescence has been induced (see Experimental Example 14 and FIG. 26D).

The oligonucleotide according to one aspect of the present invention may have cross-species efficacy. Specifically, the oligonucleotide according to one aspect of the present invention can effectively inhibit miR-204 and restore cartilage matrix synthesis function not only in mouse-derived chondrocytes but also in canine chondrocytes. The oligonucleotide according to one aspect of the present invention can exhibit miR-204 inhibitory effects regardless of species specificity.

Furthermore, the oligonucleotide according to one aspect of the present invention can be efficiently delivered without a separate nucleic acid carrier. Specifically, the oligonucleotide according to one aspect of the present invention can be efficiently delivered to chondrocytes, cartilage, and/or joint tissue without a separate nucleic acid carrier. The term "carrier-free" or "carrier-independent" used herein means that the oligonucleotide of the present invention can be introduced into cells without a separate nucleic acid carrier such as cationic lipids, lipid nanoparticles, liposomes, cationic polymers (e.g., polyethyleneimine, poly-L-lysine, etc.), dendrimers, cell-penetrating peptides, virus/virus-like particle-based vectors, and the like. The oligonucleotide according to one aspect of the present invention may be administered in a form dissolved alone in an aqueous solution (e.g., nuclease-free water (NFW), buffer solution, isotonic solution, etc.) that is substantially free of such carriers.

The oligonucleotide according to one aspect of the present invention may be formulated as an intra-articular injection and may not include a separate nucleic acid carrier except for pharmaceutically acceptable solvents (e.g., NFW, physiological saline, buffered saline, etc.) and optional stabilizers, buffers, and isotonic agents. Despite being such a carrier-free composition, the oligonucleotide of the present invention can remain stable in articular cartilage for an extended period (e.g., for several weeks after administration) while degrading miR-204 and exhibiting the effect of significantly ameliorating pathological changes of OA (see experimental examples). Therefore, the oligonucleotide of the present invention has the advantage of exhibiting excellent tissue penetration and sustained drug efficacy without requiring an expensive carrier.

The oligonucleotide according to one aspect of the present invention can be efficiently absorbed into chondrocytes (e.g., mouse-derived chondrocytes, canine chondrocytes, etc.) in cell culture systems by simply being added directly to the culture medium without separate transfection reagents (e.g., lipofectamine, cationic lipid complexes, etc.). As such, the oligonucleotide of the present invention exhibits excellent cellular uptake and miR-204 degradation ability even under carrier-free conditions, thereby providing the advantage of minimizing additional toxicity, immunogenicity, or manufacturing process complexity attributable to carriers.

Of course, as needed, the oligonucleotide according to one aspect of the present invention may be used in combination with other pharmaceutical delivery systems selected by those skilled in the art, in addition to the above-described carrier-free forms. For example, the oligonucleotide according to one aspect of the present invention may be delivered to chondrocytes, cartilage, and/or joint tissue via a carrier.

### Compositions, uses and methods for preventing or treating OA

Furthermore, one aspect of the present invention provides a composition for preventing or treating OA, including an oligonucleotide according to the foregoing aspect. Provided is a pharmaceutical composition including an oligonucleotide according to the invention. Also provided is an oligonucleotide according to the foregoing aspect for use as a medicament. In one aspect, the invention provides an oligonucleotide as herein disclosed for use in a method of ameliorating, preventing, or treating OA in a subject.

The oligonucleotide has been described above, and thus detailed description thereof is omitted.

The term "OA" as used herein refers to a degenerative joint disease characterized by degeneration of articular cartilage, subchondral bone sclerosis, osteophyte development, and synovial inflammation. Risk factors such as aging, obesity, and mechanical overload may contribute to the onset and progression of OA.

The term "treatment" as used herein refers to any act of ameliorating or beneficially modifying the symptoms of OA or diseases resulting therefrom by administration of an oligonucleotide and/or composition according to one aspect of the present invention. A person having ordinary skill in the art to which the present application pertains will be able to identify precise criteria for the disease and determine the degree of amelioration, improvement, and treatment by referring to data presented in the art.

The term "prevention" as used herein refers to any act of inhibiting or delaying the onset of OA or diseases resulting therefrom by administration of an oligonucleotide and/or composition according to one aspect of the present invention. Furthermore, the term "prevention" may include acts of reducing the risk of OA or diseases resulting therefrom, slowing the progression rate of the disease, or inhibiting the manifestation of disease symptoms.

In one embodiment, OA may be a degenerative disease in which joint cartilage normally formed at birth progressively degenerates due to acquired factors such as aging, trauma, mechanical overload, and inflammation. In one embodiment, OA includes degenerative (primary) OA, post-traumatic OA, post-surgical OA, inflammatory OA, and secondary OA. For example, degenerative OA may be the most common form of OA caused by age-related, natural degenerative changes that occur without a specific cause such as definite trauma, structural disease, or inflammatory disease. For example, post-traumatic OA may be OA that occurs when degenerative changes are accelerated following mechanical trauma such as joint injury or fracture, ligament injury or rupture, meniscal injury or rupture, or damage to joint structure. For example, inflammatory OA may be OA characterized by prominent inflammation of the synovium and surrounding tissues within the joint in addition to structural degenerative changes, and may be OA characterized by synovial inflammation or increased inflammatory cells in synovial fluid. Post-surgical OA may be OA secondarily induced by structural changes, instability, or biomechanical changes following surgical treatment of intra-articular or surrounding tissues. Secondary OA may be OA that occurs when joint structure or biological environment is altered by a definite underlying cause (trauma, congenital malformation, metabolic disease, endocrine disorder, infection, crystal deposition disease, etc.). In this case, congenital malformations may include hip dysplasia, varus/valgus deformity, and the like, and metabolic and endocrine disorders may include diabetes, thyroid disease, avascular necrosis, obesity, and the like.

The composition according to one aspect of the present invention may be for preventing or treating OA in mammals. The mammal may be at least one selected from the group consisting of a human, a dog, a cat, a rabbit, a cow, a horse, a pig, a sheep, a mouse, a rat, a monkey, and a chimpanzee, but is not limited thereto. Specifically, the mammal may be a human, a dog, or a cat.

The composition according to one aspect of the present invention may be used to alleviate various pathological changes of OA. Specifically, the composition may be used to alleviate at least one selected from the group consisting of cartilage destruction, subchondral bone sclerosis, osteophyte development, synovial inflammation, cartilage matrix loss, and/or OA-associated pain. In this regard, one aspect of the present invention may provide a composition for alleviating cartilage destruction, subchondral bone sclerosis, osteophyte development, synovial inflammation, cartilage matrix loss, and/or OA-associated pain, including the oligonucleotide according to one aspect of the present invention

The composition according to one aspect of the present invention may be used to restore the extracellular matrix (ECM) synthesis function of chondrocytes. Specifically, the composition may be used for the purpose of increasing or restoring the expression of the proteoglycan biosynthetic enzyme. Furthermore, the composition may be used for increasing or restoring the sGAG content. In this regard, one aspect of the present invention may provide a composition for increasing proteoglycan biosynthetic enzyme and/or sGAG, including the oligonucleotide according to one aspect of the present invention.

The composition according to one aspect of the present invention may be used to reduce or restore the expression of cartilage matrix degrading enzymes or to suppress or alleviate inflammation. Specifically, the composition may be used for the purpose of reducing the expression of cartilage matrix degrading enzymes, and the cartilage matrix degrading enzyme may include Mmp3, but is not limited thereto. Furthermore, the composition may be used for reducing the expression of inflammatory cytokines. The inflammatory cytokines may be IL-6, IL-1β, TNF-α, or a combination thereof, but is not limited thereto. In this regard, one aspect of the present invention may provide a composition for reducing cartilage matrix degrading enzymes and inflammatory cytokines, including the oligonucleotide according to one aspect of the present invention.

The composition according to one aspect of the present invention may be used for preventing or treating early and/or late stages of OA. The composition may restore cartilage even in the late stage when OA has already progressed. For example, the early stage of OA may be the stage of OA induction or within one, two, three, or four weeks after diagnosis, in which mild cartilage damage is observed but subchondral bone sclerosis, osteophyte development, or the like has not progressed, but is not limited thereto. For example, the late stage of OA may be the stage of OA induction or after four weeks after diagnosis, in which severe cartilage damage is observed and subchondral bone sclerosis, osteophyte development, and the like are advanced, but is not limited thereto.

The composition according to one aspect of the present invention may exhibit a superior effect compared to conventional OA therapeutic agents. Specifically, the formulation may exhibit superior effects compared to conventional OA therapeutic agents in terms of cartilage protection, cartilage recovery, cartilage regeneration, subchondral bone sclerosis amelioration, inflammation alleviation, increased sGAG synthesis, and/or pain alleviation. Conventional OA therapeutic agents may include SM04690 or dexamethasone, but are not limited thereto.

The composition according to one aspect of the present invention may be for use in animals (e.g., mammals), but is not limited thereto. For example, the animal may be at least one selected from the group consisting of a human, a dog, a cat, a rabbit, a cow, a horse, a pig, a sheep, a mouse, a rat, a monkey, and a chimpanzee, but is not limited thereto. Specifically, the animal may be a human or a dog.

The composition according to one aspect of the present invention may be a pharmaceutical composition, a food composition, a health functional food composition, or an oral composition, but is not limited thereto.

When the composition according to one aspect of the present invention is a pharmaceutical composition, the composition may additionally include pharmaceutically acceptable carriers, diluents, coagulants, stabilizers, buffers, or isotonic agents, or the like. Any pharmaceutically acceptable carrier, diluent, coagulant, stabilizer, buffer, or isotonic agent known in the art may be used without limitation, and it may be, for example, at least one selected from the group consisting of saline, sterile water, Ringer's solution, buffered saline, dextrose solution, maltodextrin solution, glycerol, ethanol, and a combination thereof, but is not limited thereto. In addition, other commonly used additives, such as antioxidants and bactericidal agents, may be additionally included as needed.

The composition according to one aspect of the present invention may not include a carrier. The composition according to one aspect of the present invention may effectively deliver the oligonucleotide according to one aspect of the present invention into cells, exhibit miR-204 degradation activity, and demonstrate OA prevention or therapeutic effects even under carrier-free conditions. Meanwhile, when necessary, the composition according to one aspect of the present invention may include a carrier. The carrier may be a cationic lipid, a lipid nanoparticle, a liposome, a cationic polymer (e.g., polyethyleneimine, poly-L-lysine, etc.), a dendrimer, a cell-penetrating peptide, a viral particle-based vector, and/or a virus-like particle-based vector, but is not limited thereto.

The composition according to one aspect of the present invention may be prepared in dosage forms. For example, the composition may be formulated in the form of an injection, a liquid, a suspension, an emulsion, a syrup, powder, a granule, a tablet, a capsule, gel, a patch, aerosol, or a combination thereof, but is not limited thereto. Specifically, the composition may be formulated as an intra-articular injection. The composition may be for intra-articular injection. For example, intra-articular injection can deliver a drug locally at a high concentration, thereby maximizing therapeutic effects while minimizing systemic side effects.

The oligonucleotide or composition according to one aspect of the present invention may be administered by various routes. For example, the composition may be administered by oral administration, parenteral administration, subcutaneous administration, intravenous administration, intramuscular administration, intraperitoneal administration, transdermal administration, nasal administration, pulmonary administration, rectal administration, topical administration (e.g., intra-articular injection, other topical injection, topical application), or intra-articular administration, but is not limited thereto. Specifically, the composition may be administered by intra-articular administration. In examples according to one aspect of the present invention, verification was carried out through intra-articular administration, but a person of ordinary skill in the art will understand that appropriate application through other administration routes is also possible considering pharmacokinetics/delivery mechanisms.

The oligonucleotide or composition according to one aspect of the present invention may be administered in a pharmaceutically effective amount. In one aspect of the present specification, the term "pharmaceutically effective amount" refers to an amount sufficient to treat a disease at a reasonable benefit/risk ratio applicable to medical treatment, and the effective dose level may be determined according to factors including the type and severity of the patient's disease, activity of the drug, sensitivity to the drug, administration time, administration route and excretion rate, treatment duration, concomitantly used drugs, and other factors well known in the medical field.

The administration amount of the oligonucleotide or composition according to one aspect of the present invention may be appropriately selected by those skilled in the art taking into account the patient's age, sex, body weight, severity of the disease, administration route, and administration frequency. For example, the amount of the oligonucleotide may be 0.1 mg to 50 mg per single dose based on an adult (e.g., a 60 kg adult). For example, based on an adult (e.g., a 60 kg adult), the oligonucleotide may be administered in a single dose of 0.1 mg or more, 1 mg or more, 5 mg or more, 9 mg or more, 10 mg or more, 11 mg or more, 15 mg or more, 20 mg or more, 30 mg or more, 40 mg or more, 50 mg or more, 0.1 mg or less, 1 mg or less, 5 mg or less, 9 mg or less, 10 mg or less, 11 mg or less, 15 mg or less, 20 mg or less, 30 mg or less, 40 mg or less, 50 mg or less, or a combination range thereof (e.g., 10 to 30 mg), but is not limited thereto. The composition may be administered once to several times per day, or may be repeatedly administered at intervals of several days, weeks, or months.

The oligonucleotide or composition according to one aspect of the present invention may be administered as a single dose or repeatedly administered. Specifically, the oligonucleotide or composition may be repeatedly administered once, twice, three times, four times, five times or more.

The composition according to one aspect of the present invention may be administered in combination with the oligonucleotide according to one aspect of the present invention and other OA therapeutic agents (e.g., commercialized OA therapeutic agents or therapeutic agents in clinical trials) simultaneously, separately, or sequentially.

When the composition according to one aspect of the present invention is a food composition or health functional food composition, the composition may be prepared in a form that may be consumed like ordinary food. There is no particular limitation on the type of the food composition, and the food composition may be, for example, meat, sausages, bread, chocolate, candy, snacks, confectioneries, pizza, ramen, other noodles, gum, dairy products including ice cream, various soups, beverages, tea, drinks, alcoholic beverages, vitamin complexes, or health functional foods, but is not limited thereto.

The composition according to one aspect of the present invention can effectively degrade miR-204 to prevent or treat OA. For example, the composition may degrade or reduce miR-204, regulate genes associated with ECM remodeling and biological pathways associated with cartilage structure maintenance, and reduce the expression of proteins associated with cartilage matrix destruction, thereby preventing or treating OA.

In addition, one aspect of the present invention provides a method for preventing or treating OA, including a step of administering the above-described oligonucleotide or composition to a subject.

In one embodiment, the subject may be one in need of treatment or prevention of OA.

In one embodiment, the subject may include a mammal, but is not limited thereto. For example, the mammal may be at least one selected from the group consisting of a human, a dog, a cat, a rabbit, a cow, a horse, a pig, a sheep, a mouse, a rat, a monkey, and a chimpanzee, but is not limited thereto. Specifically, the mammal may be a human or a dog.

In the method for preventing or treating OA according to one aspect of the present invention, the oligonucleotide or composition may be administered to the subject in an amount effective for the prevention or treatment of OA.

In addition, one aspect of the present invention provides a method for alleviating cartilage destruction, subchondral bone sclerosis, osteophyte development, synovial inflammation, cartilage matrix loss, and/or OA-associated pain, including a step of administering the above-described oligonucleotide or composition to a subject. Furthermore, one aspect of the present invention provides a method for increasing proteoglycan biosynthetic enzymes and/or sGAG, including a step of administering the above-described oligonucleotide or composition to a subject. Furthermore, one aspect of the present invention provides a method for decreasing cartilage matrix degrading enzymes and/or inflammatory cytokines, including a step of administering the above-described oligonucleotide or composition to a subject.

In addition, one aspect of the present invention provides the above-described oligonucleotide or composition for use in a method for alleviating cartilage destruction, subchondral bone sclerosis, osteophyte development, synovial inflammation, cartilage matrix loss, and/or OA-associated pain, including a step of administering the above-described oligonucleotide or composition to a subject. Furthermore, one aspect of the present invention provides the above-described oligonucleotide or composition for use in a method for increasing proteoglycan biosynthetic enzymes and/or sGAG, including a step of administering the above-described oligonucleotide or composition to a subject. Furthermore, one aspect of the present invention provides the above-described oligonucleotide or composition for use in a method for decreasing cartilage matrix degrading enzymes and/or inflammatory cytokines, including a step of administering the above-described oligonucleotide or composition to a subject.

In one embodiment, the subject may be one in need of alleviation of cartilage destruction, subchondral bone sclerosis, osteophyte development, synovial inflammation, cartilage matrix loss, and/or OA-associated pain. In one embodiment, the subject may be one in need of an increase in proteoglycan biosynthetic enzymes and/or sGAG. In one embodiment, the subject may be one in need of a decrease in cartilage matrix degrading enzymes and/or inflammatory cytokines.

In one embodiment, the oligonucleotide or composition may be administered to a subject in an amount effective for alleviating cartilage destruction, subchondral bone sclerosis, osteophyte development, synovial inflammation, cartilage matrix loss, and/or OA-associated pain. In one embodiment, the oligonucleotide or composition may be administered to a subject in an amount effective for increasing proteoglycan biosynthetic enzymes and/or sGAG. In one embodiment, the oligonucleotide or composition may be administered to a subject in an amount effective for reducing cartilage matrix degrading enzymes and/or inflammatory cytokines.

The administration route, dose, administration frequency, combined administration, and the like have been described above, so detailed descriptions thereof are omitted.

In addition, one aspect of the present invention provides use of the above-described oligonucleotide for preparing a composition for preventing or treating OA.

In addition, one aspect of the present invention provides use of the above-described oligonucleotide for preventing or treating OA.

Oligonucleotides, treatment, prevention, and OA have been described above, so detailed descriptions thereof are omitted.

In addition, one aspect of the present invention provides use of the above-described oligonucleotide for preparing a composition for alleviating cartilage destruction, subchondral bone sclerosis, osteophyte development, synovial inflammation, cartilage matrix loss, and/or OA-associated pain. Furthermore, one aspect of the present invention provides use of the above-described oligonucleotide for preparing a composition for increasing proteoglycan biosynthetic enzymes and/or sGAG. Furthermore, one aspect of the present invention provides use of the above-described oligonucleotide for preparing a composition for reducing cartilage matrix degrading enzymes and/or inflammatory cytokines.

In addition, one aspect of the present invention provides use of the above-described oligonucleotide for alleviating cartilage destruction, subchondral bone sclerosis, osteophyte development, synovial inflammation, cartilage matrix loss, and/or OA-associated pain. Furthermore, one aspect of the present invention provides use of the above-described oligonucleotide for increasing proteoglycan biosynthetic enzymes and/or sGAG. Furthermore, one aspect of the present invention provides use of the above-described oligonucleotide for reducing cartilage matrix degrading enzymes and/or inflammatory cytokines.

In addition, one aspect of the present invention provides the followings:

### [Embodiment 1]

An oligonucleotide specifically hybridizing with miR-204, wherein the oligonucleotide includes 20 to 22 linked nucleotides and consists of a first region, a second region, and a third region in order from a 5' end to a 3' end, wherein the first region includes three consecutively connected nucleotides, each complementary to a tail region of miR-204 and including a constrained sugar modification, the second region includes three connected nucleotides connected, the nucleotides included in the second region each independently being nucleotides including a constrained sugar modification or a non-constrained 2'-substituted sugar modification, and the third region includes a plurality of connected nucleotides, the nucleotides included in the third region each independently being nucleotides including a non-constrained 2'-substituted sugar modification, and wherein the oligonucleotide includes 30% or less of nucleotides including a constrained sugar modification based on a total number of nucleotides.

### [Embodiment 2]

The oligonucleotide of Embodiment 1, wherein the nucleotide including the constrained sugar modification is at least one selected from the group consisting of a constrained nucleic acid (LNA), a 2',4'-ethylene-bridged nucleic acid (ENA), constrained ethyl nucleotide (cEt), a bridged nucleic acid (BNA), and an acyclic amino-bridged BNA (BNANC).

### [Embodiment 3]

The oligonucleotide of Embodiment 1 or 2, wherein the non-constrained 2'-substituted sugar modification is at least one selected from the group consisting of 2'-deoxy, 2'-O-methyl (2'-O-Me), 2'-O-methoxyethyl (2'-O-MOE), 2'-O-alkyl, 2'-fluoro (2'-F), 2'-amino (2'-NH₂), 2'-O-allyl, 2'-O-benzyl, ribose, and arabinose (ANA) non-constrained 2'-substituted sugar modifications.

### [Embodiment 4]

The oligonucleotide of any one of Embodiments 1 to 3, wherein the nucleotides included in the third region all include a 2'-deoxy sugar.

### [Embodiment 5]

The oligonucleotide of any one of Embodiments 1 to 4, wherein sugars of the nucleotides at positions 1 and 2 from the 3' end of the oligonucleotide are 2'-deoxy.

### [Embodiment 6]

The oligonucleotide of any one of Embodiments 1 to 5, wherein the oligonucleotide includes a region complementary to a seed region of the miR-204, and sugars of nucleotides included in the region complementary to the seed region are non-constrained sugar modifications.

### [Embodiment 7]

The oligonucleotide of any one of Embodiment s 1 to 6, wherein internucleotide bonds included in the oligonucleotide are each independently a phosphorothioate bond or a phosphodiester bond.

### [Embodiment 8]

The oligonucleotide of any one of Embodiments 1 to 7, wherein the oligonucleotide is one or more of oligonucleotides represented by SEQ ID NOs: 1 to 14 and 26 to 39.

### [Embodiment 9]

The oligonucleotide of any one of Embodiments 1 to 8, wherein the second region or the third region includes one or more mismatch nucleotides with the miR-204.

### [Embodiment 10]

The oligonucleotide of any one of Embodiments 1 to 9, wherein the oligonucleotide is an oligonucleotide for degrading the miR-204.

### [Embodiment 11]

A use of the oligonucleotide of any one of Embodiments 1 to 10 for ameliorating, preventing, or treating osteoarthritis.

### [Embodiment 12]

The use of Embodiment 11, wherein the osteoarthritis is at least one selected from the group consisting of degenerative osteoarthritis, post-traumatic osteoarthritis, post-surgical osteoarthritis, inflammatory osteoarthritis, and secondary osteoarthritis.

### [Embodiment 13]

The use of Embodiment 11 or 12, wherein the subject is a mammal.

### [Embodiment 14]

The oligonucleotide of any one of Embodiments 1 to 10 for use in a method for ameliorating, preventing, or treating osteoarthritis.

### [Embodiment 15]

The oligonucleotide for use of Embodiment 14, wherein the osteoarthritis is at least one selected from the group consisting of degenerative osteoarthritis, post-traumatic osteoarthritis, post-surgical osteoarthritis, inflammatory osteoarthritis, and secondary osteoarthritis.

### [Embodiment 16]

The oligonucleotide for use of Embodiment 14 or 15, wherein the subject is a mammal.

Hereinafter, the components and effects of the present invention will be described in more detail through examples and experimental examples. However, the following examples are provided only for illustrative purposes to aid understanding of the present invention, and the scope and range of the present invention are not limited thereby.

### Description of Government-Supported Research Project

[National Research and Development Project Supporting This Invention]
[Project Identification Number] 2420003227. [Project Number] RS-2024-00437503
[Ministry] Ministry of SMEs and Startups. [Project Management (Specialized) Agency] Korea Technology Information Promotion Agency for SMEs
[Research Project Name] Startup Growth Technology Development. [Research Project Name] Development of a Next-Generation Osteoarthritis Treatment Based on MicroRNA Degraders. [Project Implementing Agency] LIFLEX SCIENCE INC.

### [Preparation Example] Preparation of miR-204-specific ASO

Oligonucleotides specific to miR-204, specifically ASOs, were prepared by the method described below (Example 1 and Example 2).

Example 1 is a 22-mer ASO consisting of the sequence 5'-+A+G+G+C+A+TAGGATGA/iMe-dC/AAAGGGAA-3' (SEQ ID NO: 1). Example 2 is a 22-mer ASO consisting of the sequence 5'-+A*+G*+G*+C*+A*+T*A*G*G*A*T*G*A*/iMe-dC/*A*A*A*G*G*G*A*A-3' (SEQ ID NO: 2). In this specification, capital letters represent DNA bases, "+X" represents LNA bases, and "iMe-dC" represents 5-methyl-deoxycytosine bases. In addition, the "*" symbol indicates that bases are linked by a PO bond, while the absence of the "*" symbol indicates that bases are linked by a PS bond.

The ASO was synthesized and purified by a contract manufacturing organization (CMO) and supplied in powder form. The supplied material was dissolved in nuclease-free water (NFW; AM9932, Thermo Fisher Scientific) to prepare a solution at a concentration of 5 mM, and the resulting solution was stored at -80 °C.

For cell experiments, the material was thawed at room temperature immediately before use, then diluted in culture medium to the target concentration and applied to cells. For animal experiments, the material was thawed at room temperature immediately before use, then diluted in NFW to the target concentration and injected intra-articularly in a final volume of 5 µL.

### [Experimental Example 1] Confirmation of miR-204 degradation activity of ASO specific to miR-204

### [Experimental Example 1-1] Confirmation of miR-204 degradation activity of ASO of Example 1

### (1) Experimental method

C28/I2 cell lines (chondrocyte cell lines) overexpressing microRNA-204 (miR-204) were plated into 12-well plates at a density of 3 × 10⁵ cells/well and cultured for 24 hours. The cells were maintained at 37 °C under 5% CO₂ and atmospheric oxygen concentration conditions, and the culture medium used was Dulbecco's Modified Eagle Medium/Nutrient Mixture F-12 (DMEM/F-12) medium supplemented with 10% fetal bovine serum (FBS) and 1% antibiotic (penicillin-streptomycin).

After 24 hours of culture, the ASO of Example 1 was diluted in the culture medium at concentrations of 0.001, 0.01, 0.1, 0.316, 1, 3.16, and 10 µM, and applied to the cells. To this end, the existing culture medium was removed and replaced with fresh medium containing ASO to treat the cells.

Forty eight hours after ASO treatment of Example 1, cells were harvested, and total RNA was extracted using TRI Reagent (Molecular Research Center, Inc.). The extracted RNA was synthesized into cDNA using miRCURY LNA RT kit (Qiagen N.V.). The miR-204 expression levels of the synthesized cDNA were analyzed through quantitative reverse transcription polymerase chain reaction (qRT-PCR) using miRCURY LNA microRNA PCR Kit (Qiagen N.V.). Let-7e-5p (miRCURY LNA^{™} miRNA PCR assay, Qiagen N.V.) was used as an internal control. The maximum degradation efficacy (Dₘₐₓ) and the half-maximal degradation concentration (DC₅₀) of miR-204 of Example 1 were calculated using GraphPad Prism (Dotmatics).

### (2) Experimental results

Since miR-204 is present at a very low basal level in normal chondrocytes, a chondrogenic cell line (C28/I2) overexpressing miR-204 was used to sensitively evaluate the miR-204 degradation efficacy of Example 1. As a result of treating the cell line with Example 1 at various concentrations, it was confirmed that miR-204 expression decreased in a dose-dependent manner in the Example 1 treatment group (FIG. 1A). A quantitative analysis showed that the maximum degradation efficacy (Dₘₐₓ) of Example 1 was approximately 99.6%, and the reaction concentration for 50% degradation (DC₅₀) of miR-204 was calculated to be approximately 0.0055 µM (FIG. 1B).

### [Experimental Example 1-2] Confirmation of miR-204 degradation ability of ASO of Example 2

### (1) Experimental method

C28/I2 chondrocytes overexpressing miR-204 were cultured in a 12-well plate under conditions of 5% CO₂, atmospheric oxygen, and 37 °C using DMEM/F-12 medium supplemented with 10% FBS and 1% penicillin-streptomycin. A portion of the cells were seeded at 3 × 10⁵ cells/well and cultured for 24 hours, after which the culture medium was removed and 1 µM of Example 2 compound along with a transfection reagent, MessengerMAX reagent, and Opti-MEM were diluted and incubated at room temperature for 10 minutes. Thereafter, cells in a 0.5 ml of fresh medium were treated with Example 2 diluted with the transfection reagent and Opti-MEM, and then the medium was exchanged with fresh medium after six hours. The cells were harvested 48 hours later (FIG. 14). Total RNA was extracted from the harvested cells using TRI Reagent (Molecular Research Center, Inc.), cDNA was synthesized using miRCURY LNA RT Kit (Qiagen), and miR-204 expression was analyzed by qRT-PCR using miRCURY LNA microRNA PCR Kit (Qiagen), with Let-7e-5p used as an internal control.

For statistical analysis, all quantitative data were expressed as mean ± standard error of the mean (Mean ± SEM). Statistical significance was tested using GraphPad Prism 10 software (version 10.4.2, GraphPad Software) with Student's t-test. Statistical significance indicators in the graphs are as follows: *, **, ***, ****: P < 0.05, 0.01, 0.001, 0.0001, respectively.

### (2) Experimental results

The qRT-PCR analysis showed that the mean expression level of miR-204 in the vehicle trf was 1.000 (SEM: 0.000), whereas in Example 2, specifically, the mean expression of miR-204 with transfected (carrier-loaded) oligonucleotide (0.1 µM) was 0.323 (SEM: 0.030), confirming that the ASO including the PO backbone also induced degradation of miR-204 (see FIG. 14).

This degradation effect of miR-204 suggests that when an oligonucleotide (ASO) according to one aspect of the present invention is delivered into cells, the target miR-204 can be effectively degraded regardless of the backbone components.

### [Experimental Example 2] Confirmation of mouse chondrocyte delivery of miR-204-specific ASO (Example 1)

### (1) Experimental method

### 1) Primary culture of mouse chondrocytes

Mouse articular chondrocytes were isolated from the femoral condyles and tibial plateaus of 5 to 6 day-old Institute of Cancer Research (ICR) mice. The isolated tissue was enzymatically digested using 0.2% collagenase solution (ref) to obtain a single cell suspension. The obtained cells were plated into 12-well plates at a density of 2 × 10⁵ cells/well for flow cytometry in DMEM supplemented with 10% FBS (Gibco) and 1% penicillin-streptomycin (Sigma-Aldrich). The cells were maintained at 37 °C, 5% CO₂ and 21% O₂. At 72 hours after cell plating, ASO treatment groups and vehicle control groups were established according to each experimental purpose. For fluorescence imaging, cells were seeded in 12-well plates at a density of 1 × 10⁵ cells/well and cultured. The cells were maintained at 37 °C, 5% CO₂ and 21% O₂. At 96 hours after cell plating, ASO treatment groups and vehicle control groups were established according to each experimental purpose.

### 2) Confirmation of delivery efficiency of the ASO of Example 1

To visualize the cellular uptake pattern of Example 1 in chondrocytes, cultured cells were incubated with 1 µM Cy5.5 fluorescently-labeled Example 1 for six hours. Thereafter, Hoechst at a concentration of 1 µg/ml was added to the culture medium and co-incubated for an additional 18 hours. For fluorescence microscopy analysis, cells were washed three times with phosphate-buffered saline (PBS), and fluorescence images were acquired using the EVOS M7000 Imaging System (Thermo-Fisher, AMF7000).

In addition, a flow cytometry analysis was performed to quantitatively evaluate the degree of cellular uptake of Example 1. To this end, mouse articular chondrocytes were cultured with Cy3 fluorescently-labeled Example 1 (10 nM or 50 nM) for 24 hours. After completion of treatment, the cells were detached from the culture surface using trypsin, washed with PBS, and then recovered by centrifugation. The recovered cells were resuspended twice in a PBS solution containing 5% FBS.

A flow cytometry analysis was performed using FACSAria^{™} III Cell Sorter (BD Biosciences) equipment, and analytical data were collected and quantitatively analyzed using FACSDiva 9.7 software.

### (2) Experimental results

To confirm the delivery efficiency of Example 1 to mouse-derived chondrocytes, cellular uptake was evaluated using Cy5.5 or Cy3 fluorescently-labeled Example 1. As a result of fluorescence microscopy analysis, it was confirmed that fluorescent signals were distinctly accumulated intracellularly in mouse-derived chondrocytes of the Example 1 treatment group compared to the vehicle treatment group (FIG. 2A). Furthermore, as a result of the flow cytometry analysis, fluorescent positive signals of more than 50% were observed under all conditions treated with Example 1 at concentrations of 10 nM and 50 nM, quantitatively confirming the excellent cellular uptake capability of Example 1 (FIGS. 2B and 2C). These results suggest that Example 1 has physicochemical properties that enable efficient delivery into chondrocytes and stable accumulation therein.

### [Experimental Example 3] Confirmation of delivery capability of Example 1 in OA patient tissue

### (1) Experimental method

### 1) In vitro culture of human cartilage tissue

OA patient tissues obtained from joint replacement surgery were cultured in DMEM supplemented with 10% FBS (Gibco) and 1% penicillin-streptomycin (Sigma-Aldrich). The tissue were maintained at 37 °C, 5% CO₂, and 21% O₂. ASO treatment groups and vehicle control groups were established according to each experimental purpose.

### 2) Confirmation of delivery of Example 1

To visualize the cellular uptake pattern of Example 1 in chondrocytes, cultured cells were incubated with Cy5.5 fluorescently-labeled Example 1 at a concentration of 1 µM for 24 hours. Thereafter, Hoechst at a concentration of 1 µg/ml was added to the culture medium and co-incubated for an additional two hours. For fluorescence microscopy analysis, the cells were washed three times with PBS, and fluorescence images were acquired using an EVOS M7000 Imaging System (Thermo-Fisher, AMF7000).

### (2) Experimental results

To confirm delivery to OA patient-derived cartilage tissue in Example 1, cellular uptake was evaluated using Cy5.5 fluorescently-labeled Example 1. As a result of fluorescence microscopy analysis, it was confirmed that fluorescent signals were distinctly accumulated intracellularly in patient-derived chondrocytes treated with Example 1 compared to the vehicle treatment group (FIG. 3). These results suggest that Example 1 has physicochemical properties that enable efficient delivery and stable accumulation not only in mouse chondrocytes but also in human chondrocytes.

### [Experimental Example 4] Confirmation of miR-204 degradation activity of miR-204-specific ASO

To confirm the miR-204 degradation activity of the full DNA ASO having the same base sequence as the ASO of Example 1 prepared in the above Preparation Example but composed entirely of DNA, and ASOs having partially different base sequences, that is, ASOs including mismatches (indicated in bold) (Examples 3-1 to 3-3, Table 1 below), the following experiment was performed.

Here, the full DNA refers to an ASO having a base sequence completely complementary to target miR-204, and sugars of the nucleotides included in the full DNA ASO are all 2'-deoxy, not including LNA sugars and modified sugars.

**[Table 1]**

| Classification | Sequence (5'-3') | SEQ ID NO |
|---|---|---|
| Full DNA | AGGCATAGGATGA/iMe-dC/AAAGGGAA | 40 |
| Example 3-1 | +A+G+G+C+A+TAGGATGAGAAAGGGAA | |
| Example 3-2 | +A+G+G+C+A+TAGGAAGA/iMe-dC/AAAGGGAA | |
| Example 3-3 | +A+G+G+C+A+TACGAAGA/iMe-dC/AAAGGGAA | |

### (1) Experimental method

Experiments were carried out to analyze the expression level of miR-204 by qRT-PCR in the same method as in Experimental Example 1 (however, the treatment concentrations in Examples 1 and 3-1 to 3-3 were 1 µM and 10 µM).

For statistical analysis, all quantitative data were expressed as Mean ± SEM. Statistical significance was tested using GraphPad Prism 10 software (version 10.4.2, GraphPad Software), and Tukey's post-hoc test was applied after performing one-way ANOVA. Statistical significance indicators in the graphs are as follows:
*, **, ***, ****: Comparison with vehicle (P < 0.05, 0.01, 0.001, 0.0001, respectively);
#, ##, ###, ####: Comparison of ASO (1 µM concentration group) with the full DNA (P < 0.05, 0.01, 0.001, 0.0001, respectively);
$, $$, $$$, $$$$: Comparison of ASO (10 µM concentration group) with the full DNA (P < 0.05, 0.01, 0.001, 0.0001, respectively);

### (2) Experimental results

The qRT-PCR analysis showed that miR-204 expression was statistically significantly decreased in the Example 3-1 to 3-3 treatment groups compared to the control group (Full DNA) without LNA (see Table 2, FIG. 4).

**[Table 2]**

| Classification | No. | Mean miR-204 expression level | | SEM | |
|---|---|---|---|---|---|
| | | 1 µM | 10 µM | 1 µM | 10 µM |
| Vehicle | | 1.000 | | 0.000 | |
| Full DNA | | 1.357 | 0.498 | 0.182 | 0.110 |
| Example | 3-1 | 0.277 | 0.031 | 0.073 | 0.011 |
| | 3-2 | 0.552 | 0.093 | 0.220 | 0.015 |
| | 3-3 | 0.542 | 0.193 | 0.137 | 0.038 |

At 1 µM treatment conditions, Examples 3-1 to 3-3 all showed significant expression reduction compared to the full DNA, and the same trend was maintained at 10 µM treatment conditions.

In particular, Examples 3-1 and 3-2 each included one mismatch in the central region (nucleotides 9 to 12) of miR-204, and Example 3-3 included two mismatches in the supplementary region (nucleotides 13 to 16), but maintained miR-204 degradation activity. These results suggest that single or multiple mismatches in the central region or supplementary region of miR-204 do not significantly affect the binding efficiency of the ASO, and that complete complementarity at these sites is not necessary.

Therefore, it is determined that ASO variants (Examples 3-1 to 3-3) including one or more mismatches in the central region or supplementary region of miR-204 maintain excellent stability, binding affinity, and degradation induction efficacy for miR-204.

### [Experimental Example 5] Confirmation of miR-204 degradation activity according to ASO length

To confirm the miR-204 degradation activity of according to ASO length, experiments were performed using the same method as in Experimental Example 1 on shorter ASOs (Examples 4-1 and 4-2, Table 3 below) in which some base sequences in the region near the 3' end were truncated from the ASO of Example 1 prepared in the Preparation Example above.

**[Table 3]**

| Example | Sequence (5'-3') | SEQ ID NO |
|---|---|---|
| 4-1 | +A+G+G+C+A+TAGGATGA/iMe-dC/AAAGGGA | |
| 4-2 | +A+G+G+C+A+TAGGATGA/iMe-dC/AAAGGG | |

### (1) Experimental method

An experiment was performed to analyze the expression level of miR-204 following the treatments of Examples 4-1 and 4-2 through qRT-PCR using the same method as in Experimental Example 4 above. The statistical significance indicators in the graph are as follows.
*, **, ***, ****: Comparison with vehicle (P < 0.05, 0.01, 0.001, 0.0001, respectively);
#, ##, ###, ####: Comparison of ASO (1 µM concentration group) with the full DNA (P < 0.05, 0.01, 0.001, 0.0001, respectively);
$, $$, $$$, $$$$: Comparison of ASO (10 µM concentration group) with the full DNA (P < 0.05, 0.01, 0.001, 0.0001, respectively).

### (2) Experimental results

The qRT-PCR analysis showed that miR-204 expression was statistically significantly decreased in both ASO treatment groups (Examples 4-1 and 4-2) having one (n-1) and two (n-2) bases truncated from the 5' end of the complementary sequence to miR-204, compared to the sequence without LNA fully complementary to miR-204 (Full DNA) (see Table 4, FIG. 5).

**[Table 4]**

| Classification | No. | Mean miR-204 expression level | | SEM | |
|---|---|---|---|---|---|
| | | 1 µM | 10 µM | 1 µM | 10 µM |
| Vehicle | | 1.000 | | 0.000 | |
| Full DNA | | 1.357 | 0.498 | 0.182 | 0.110 |
| Example | 4-1 | 0.239 | 0.097 | 0.033 | 0.028 |
| | 4-2 | 0.198 | 0.028 | 0.026 | 0.004 |

At both concentration conditions of 1 µM and 10 µM, Examples 4-1 and 4-2 maintained low miR-204 expression or even showed improved tendencies compared to the full DNA. In particular, Example 4-2 (n-2) exhibited the lowest miR-204 expression levels under both concentration conditions, confirming that miR-204 target binding and degradation induction were efficiently maintained even when the number of bases was shortened by two.

These results suggest that in antisense design of miR-204, effective binding and degradation induction can be induced even in truncated forms where a portion at the n-1 or n-2 level is truncated, even without a complete complementary sequence.

Therefore, ASO variants (Examples 4-1 and 4-2) with 1 or 2 base truncations compared to the complete complementary sequence of miR-204 are considered candidates capable of significantly degrading miR-204.

### [Experimental Example 6] Confirmation of the effect of LNA position on miR-204 degradation activity of ASO

In Experimental Examples 1 to 4 above, it was confirmed that the ASO of Example 1 exhibited excellent miR-204 degradation activity. Since the ASO of Example 1 has six consecutively linked LNAs within the first region, the following experiments were performed to determine whether the miR-204 degradation activity of the ASO is affected by the position of the LNA.

### [Experimental Example 6-1] Effect of LNA position when LNA is included at the 5' end of ASO

First, ASOs including LNA at the 5' end of the ASO but with different numbers and positions of LNA (Examples 5-1 to 5-7, and Comparative Examples 1-1 and 1-2, Table 5 below) were prepared by the same method as in the Preparation Example above, and their miR-204 degradation ability was evaluated.

**[Table 5]**

| Classification | No. | Sequence (5'→3') | SEQ ID NO |
|---|---|---|---|
| Example | 5-1 | +A+G+G/iMe-dC/+A+TAGGATGA/iMe- dC/AAAGGGAA | |
| | 5-2 | +A+G+G+CA+TAGGATGA/ime-dC/AAAGGGAA | |
| | 5-3 | +A+G+G+C+ATAGGATGA/ime-dC/AAAGGGAA | 10 |
| | 5-4 | +A+G+G/iMe-dC/A+TAGGATGA/iMe- dC/AAAGGAA | 11 |
| | 5-5 | +A+G+G+CATAGGATGA/iMe-dC/AAAGGGAA | 12 |
| | 5-6 | +A+G+G/iMe-dC/+ATAGGATGA/iMe- dC/AAAGGAA | 13 |
| | 5-7 | +A+G+G/iMe-dC/ATAGGATGA/iMe- dC/AAAGGAA | 14 |
| Comparative Example | 1-1 | +AGG/iMe-dC/ATAGGATGA/iMe-dC/AAAGGGAA | 15 |
| | 1-2 | +A+GG/iMe-dC/ATAGGATGA/iMe- dC/+AAAGGGAA | 16 |

### (1) Experimental Method

In the same method as in Experimental Example 4 above, an experiment was performed to analyze the expression level of miR-204 through qRT-PCR following the treatments of Examples 5-1 to 5-7, and Comparative Examples 1-1 and 1-2. At this time, the statistical significance indicators in the graph are as follows.

Statistical significance indicators in the graph are as follows:
*, **, ***, ****: Comparison with vehicle (P < 0.05, 0.01, 0.001, 0.0001, respectively);
#, ##, ###, ####: In Examples 5-1 to 5-7, ASO (1 µM concentration group) compared with Full DNA / In Comparative Examples 1-1 to 1-2, ASO (1 µM concentration group) compared with Example 1 (P < 0.05, 0.01, 0.001, 0.0001, respectively);
$, $$, $$$, $$$$: In Examples 5-1 to 5-7, ASO (10 µM concentration group) compared with Full DNA / In Comparative Examples 1-1 to 1-2, ASO (10 µM concentration group) compared with Example 1 (P < 0.05, 0.01, 0.001, 0.0001, respectively).

### (2) Interpretation of experimental results

qRT-PCR analysis results showed that compared to the control group without LNA (Full DNA), both ASOs with three consecutive LNAs positioned at the 5' end (wherein all three nucleotides in the first region are LNAs) (Examples 5-7) and ASOs with one or two LNAs positioned at nucleotide positions 4 to 6 from the 5' end (wherein at least one of the three nucleotides in the second region is LNA) (Examples 5-1 to 5-6) exhibited statistically significant reduction in miR-204 expression level (see Table 6 below and FIGS. 6 and 7).

**[Table 6]**

| Classification | No. | Mean miR-204 expression level | | SEM | |
|---|---|---|---|---|---|
| | | 1 µM | 10 µM | 1 µM | 10 µM |
| Vehicle | | 1.000 | | 0.000 | |
| Full DNA | | 1.357 | 0.498 | 0.182 | 0.110 |
| Example | 5-1 | 0.215 | 0.046 | 0.017 | 0.010 |
| | 5-2 | 0.248 | 0.069 | 0.036 | 0.022 |
| | 5-3 | 0.500 | 0.112 | 0.156 | 0.043 |
| | 5-4 | 0.190 | 0.143 | 0.011 | 0.038 |
| | 5-5 | 0.324 | 0.157 | 0.046 | 0.040 |
| | 5-6 | 0.218 | 0.120 | 0.024 | 0.046 |
| | 5-7 | 0.260 | 0.140 | 0.048 | 0.027 |
| | | 0.346 | 0.058 | 0.095 | 0.026 |
| Comparative Example | 1-1 | 0.474 | 0.493 | 0.118 | 0.049 |
| | 1-2 | 0.464 | 0.292 | 0.102 | 0.105 |

At both treatment concentrations of 1 µM and 10 µM, all ASOs of Examples 5-1 to 5-7 exhibited significant miR-204 degradation effects compared to the full DNA, and particularly Examples 5-1 to 5-7 showed the same tendency.

These results suggest that, for miR-204 target binding, it is possible to include LNA at the 5' end (first region, nucleotides 1 to 3) while positioning LNA or DNA at the second region (nucleotides 4 to 6 from the 5' end) and still maintain excellent miR-204 degradation activity in such cases.

Therefore, it is determined that when the LNA positions are concentrated near the 5' end (first region), an ASO with DNA or LNA mixed and positioned in the adjacent second region has stable and efficient miR-204 degradation efficacy.

### [Experimental Example 6-2] Effect of LNA position when LNA is included at the 3' end in ASO

In Experimental Example 6-1, it was confirmed that ASOs having three or more LNA nucleotides among nucleotides 1 to 6 at the 5' end of the ASO, specifically ASOs in which at least all of nucleotides 1 to 3 are LNA, exhibited excellent miR-204 degradation activity. Accordingly, to determine whether there is a difference in miR-204 degradation activity when the ASO includes LNA at the 3' end rather than the 5' end (Comparative Examples 2-1 to 2-4, Table 7 below), the miR-204 degradation activity was evaluated using the same method as in Experimental Example 1 above.

**[Table 7]**

| Classification | No. | Sequence (5'→3') | SEQ ID NO |
|---|---|---|---|
| Comparative Example | 2-1 | AGG/iMe-dC/ATAGGATGA/iMe- dC/AAAGGGA+A | 17 |
| | 2-2 | AGG/iMe-dC/ATAGGATGA/iMe- dC/AAAGGG+A+A | 18 |
| | 2-3 | AGG/iMe-dC/ATAGGATGA/iMe- dC/AAAG+G+G+A+A | 19 |
| | 2-4 | AGG/iMe-dC/ATAGGATGA/iMe- dC/AA+A+G+G+G+A+A | 20 |

### (1) Experimental method

An experiment was performed to analyze the expression level of miR-204 following the treatments of Comparative Examples 2-1 to 2-4 through qRT-PCR in the same method as in Experimental Example 4 above. At this time, the statistical significance indicators in the graph are as follows.
*, **, ***, ****: Comparison with vehicle (P < 0.05, 0.01, 0.001, 0.0001, respectively);
#, ##, ###, ####: Comparison of ASO (1 µM concentration group) with Example 1 (P < 0.05, 0.01, 0.001, 0.0001, respectively);
$, $$, $$$, $$$$: Comparison of ASO (10 µM concentration group) with Example 1 (P < 0.05, 0.01, 0.001, 0.0001, respectively).

### (2) Interpretation of Experimental Results

The effect of the terminal position of LNA on miR-204 degradation efficacy was evaluated through qRT-PCR analysis. Example 1, in which LNA was positioned at the 5' end, was compared with ASO variants in which LNA was positioned at the 3' end (Comparative Examples 2-1 to 2-4). As a result, the ASOs with LNA positioned at the 3' end (Comparative Examples 2-1 to 2-4) showed reduced miR-204 degradation efficacy compared to Example 1 with LNA positioned at the 5' end at both concentrations (1 µM and 10 µM) (see Table 8 below and FIG. 8).

**[Table 8]**

| Classification | No. | Mean miR-204 expression level | | SEM | |
|---|---|---|---|---|---|
| | | 1 µM | 10 µM | 1 µM | 10 µM |
| Vehicle | | 1.000 | 1.060 | 0.000 | 0.063 |
| Example | | 0.442 | 0.084 | 0.077 | 0.028 |
| Comparative Example | 2-1 | 0.848 | 0.705 | 0.065 | 0.054 |
| | 2-2 | 0.808 | 0.680 | 0.066 | 0.075 |
| | 2-3 | 0.532 | 0.399 | 0.086 | 0.059 |
| | 2-4 | 0.646 | 0.466 | 0.141 | 0.030 |

Under the 1 µM treatment condition, Comparative Examples 2-1 and 2-2 showed a statistically significant decrease in miR-204 degradation effect compared to Example 1, and Comparative Examples 2-3 and 2-4 showed a decreasing tendency. Under the 10 µM treatment condition, a statistically significant decrease in degradation effect was confirmed in Comparative Examples 2-1, 2-3, and 2-4, and Comparative Example 2-2 also showed a similar decreasing tendency.

These results suggest that the position of LNA has a determining effect on the miR-204 degradation activity of the ASO, and that the best efficacy is exhibited when LNA is positioned at the 5' end. In other words, when LNA is moved to the 3' end, the binding affinity of the ASO and RNase H-mediated degradation efficiency are reduced, thereby decreasing the overall miR-204 degradation effect.

### [Experimental Example 6-3] Effect of LNA position when LNA is randomly included in ASO

From Experimental Example 6-2 above, it was found that even when the ASO includes three or more LNAs, when they are included only at the 3' end, the miR-204 degradation effect is not significant. Accordingly, the following experiment was performed to evaluate the miR-204 degradation activity when LNA is randomly included within the ASO (Comparative Examples 3 and 4, Table 9 below).

**[Table 9]**

| Classification | No. | Sequence (5'→3') | SEQ ID NO |
|---|---|---|---|
| Comparative Example | | +AG+GAT+G+ACA+AA+GGG+A | 21 |
| | | +CAT+AGGA+TGA+CAAA+GGG+A | 22 |

### (1) Experimental method

C28/I2 chondrocytes overexpressing miR-204 were cultured in 12-well plates under conditions of 5% CO₂, atmospheric oxygen, and 37 °C using DMEM/F-12 medium supplemented with 10% FBS and 1% penicillin-streptomycin. A portion of the cells were seeded at 3 × 10⁵ cells/well and cultured for 24 hours, after which the culture medium was removed and replaced with fresh medium containing Comparative Example 3 diluted to 10 µM for a single treatment, and the cells were harvested 48 hours after treatment (Group 1, FIG. 9). Meanwhile, the remaining cells were seeded at 2 × 10⁴ cells/well and cultured for 24 hours, and while replacing the culture medium with fresh medium, and the cells were treated with Comparative Example 4 at concentrations of 256 nM, 1.6 µM, and 10 µM. Thereafter, second treatment was additionally performed at the same concentrations 72 hours after the initial treatment, and the cells were harvested 48 hours after the second treatment (Group 2, FIG. 10). Total RNA was extracted from the harvested cells from each group using TRI Reagent (Molecular Research Center, Inc.), cDNA was synthesized using the miRCURY LNA RT Kit (Qiagen N.V.), and miR-204 expression was analyzed by qRT-PCR using the miRCURY LNA microRNA PCR Kit (Qiagen N.V.), with Let-7e-5p used as an internal control.

For statistical analysis, all quantitative data were expressed as Mean ± SEM. Statistical significance was tested using GraphPad Prism 10 software (version 10.4.2, GraphPad Software), and Tukey's post-hoc test was applied after performing one-way ANOVA. Statistical significance indicators in the graphs are as follows:
*, **, ***, ****: P < 0.05, 0.01, 0.001, 0.0001, respectively

### (2) Interpretation of experimental results

The qRT-PCR analysis showed that Comparative Example 3, in which LNA was randomly included in the first group, exhibited a statistically significantly lower miR-204 degradation effect compared to Example 1 (see Table 10 below and FIG. 9).

**[Table 10]**

| Classification | No. | Mean miR-204 expression level | SEM |
|---|---|---|---|
| | | 10 µM | 10 µM |
| Vehicle | | 1.000 | 0.000 |
| Full DNA | | 1.0596 | 0.114 |
| Example | | 0.2776 | 0.045 |
| Comparative Example | | 0.7439 | 0.157 |

This low miR-204 degradation effect indicates that the position and arrangement pattern of LNA within the ASO is a more important factor for miR-204 degradation activity than the mere inclusion of LNA in the ASO. Similarly, Comparative Example 4, which represents another form with randomly included LNA, also showed lower miR-204 degradation activity compared to Example 1 across all concentration groups (see FIG. 10). In other words, in structures where LNA is randomly positioned as in Comparative Examples 3 and 4, efficient binding to and degradation induction of miR-204 is difficult, suggesting that the ASO according to one aspect of the present invention, such as Example 1 with its structure arranged centered around the 5' end (centered around the first and second regions), is more effective for miR-204 degradation.

### [Experimental Example 7] Confirmation of the effect of the number of LNAs on miR-204 degradation activity of ASO

In Experimental Example 6 above, it was confirmed that the miR-204 degradation activity varied depending on the position of LNA within the ASO. Therefore, to confirm the effect according to the number of LNAs within the ASO, ASOs having 15 or more LNAs positioned from the 5' end (Comparative Examples 5-1 to 5-3, Table 11 below) were prepared from the ASO of Example 1 above, and their formulation stability was evaluated.

**[Table 11]**

| Classification | No. | Sequence (5'→3') | SEQ ID NO |
|---|---|---|---|
| Comparative Example | 5-1 | +A+G+G+C+A+T+A+G+G+A+T+G+A+C+AAA GGGAA | 23 |
| | 5-2 | +A+G+G+C+A+T+A+G+G+A+T+G+A+C+A+A+ A+G+GGAA | 24 |
| | 5-3 | +A+G+G+C+A+T+A+G+G+A+T+G+A+C+A+A+ A+G+G+G+A+A | 25 |

### (1) Experimental method

In the same method as in Experimental Example 4, an experiment was performed to analyze the expression level of miR-204 following treatment with Comparative Examples 5-1 to 5-3 through qRT-PCR. The statistical significance indicators in the graph are as follows:
*, **, ***, ****: Comparison with Vehicle (P < 0.05, 0.01, 0.001, 0.0001, respectively).

In addition, to confirm the physical properties (viscosity) of the dissolved ASOs of Comparative Examples 5-1 to 5-3, tubes containing each solution were photographed obliquely from below, and the solutions were collected using a 1-ml pipet with a 1-ml pipet tip, after which the solution present in each tip was photographed.

### (2) Interpretation of experimental results

The effect of the number of LNAs in the ASO on the miR-204 degradation effect was evaluated through qRT-PCR analysis. Experiments were performed on variants with progressively increased numbers of LNAs (Comparative Examples 5-1 to 5-3), and as a result, the miR-204 degradation effect remained similar even when the number of LNAs increased (see FIG. 11A).

However, variants with excessively increased numbers of LNAs (Comparative Examples 5-2 and 5-3) exhibited a sharp increase in viscosity during the dissolution process, and aggregation of the solution into a gel form was observed (see FIG. 11B). Even a variant with a relatively few number of LNAs (Comparative Example 5-1) showed higher viscosity than Example 1. This is considered to be a phenomenon in which interactions between ASOs are enhanced as LNA content increases, resulting in decreased water solubility.

Summarizing these results, it can be seen that increasing the number of LNAs enhances the miR-204 degradation effect, but may simultaneously cause physical and formulation limitations as a drug due to increased formulation viscosity and decreased solubility. Therefore, when considering both efficacy and formulation stability, a non-excessive number of LNAs (e.g., the level of Example 1) is determined to be the optimal structure that maintains a balance between miR-204 inhibition efficacy and formulation suitability.

### [Experimental Example 8] Effects of modified sugars other than LNA on miR-204 degradation activity

In Experimental Example 6-1 above, it was confirmed that ASOs in which at least three of nucleotides 1 to 6 at the 5' end of the ASO are LNA (ASOs in which at least three of the nucleotides in the first region and second region are LNA), specifically ASOs in which all of at least nucleotides 1 to 3 are LNA (all 3 nucleotides in the first region are LNA), exhibited excellent miR-204 degradation activity. As in Experimental Example 6-1, miR-204 degradation activity was evaluated to determine whether there is a difference in miR-204 degradation activity when at least three of nucleotides 1 to 6 at the 5' end of the ASO are LNA, specifically when at least all of nucleotides 1 to 3 are LNA, but modified sugars other than LNA modified sugars (e.g., non-bicyclic 2'-substituted sugar modifications) are additionally present at other positions (Examples 6-1 to 6-14, Table 12 below; MOE modifications are indicated as X_{M}, OME modifications are indicated as Xo (X is a nucleotide)).

**[Table 12]**

| Classification | No. | Type of sugar modification | Sequence (5'→3') | SEQ ID NO |
|---|---|---|---|---|
| Example | 6-1 | MOE | +A+G+GC_M+A+TAGGATGA/iMe- dC/AAAGGAA | 26 |
| | 6-2 | | +A+G+G+CA_{M}+TAGGATGA/iMe- dC/AAAGGGAA | 27 |
| | 6-3 | | +A+G+G+C+AT_{M}AGGATGA/iMe- dC/AAAGGAA | 28 |
| | 6-4 | | +A+G+GC_{M}A_{M}+TAGGATGA/iMe- dC/AAAGGGAA | 29 |
| | 6-5 | | +A+G+G+CA_{M}T_{M}AGGATGA/iMe- dC/AAAGGGAA | 30 |
| | 6-6 | | +A+G+GCM+ATMAGGATGA/iMe- dC/AAAGGGAA | 31 |
| | 6-7 | | +A+G+GC_MA_MT_MAGGATGA/iMe- dC/AAAGGGAA | 32 |
| | 6-8 | OME | +A+G+GCo+A+TAGGATGA/iMe- dC/AAAGGAA | 33 |
| | 6-9 | | +A+G+G+CAo+TAGGATGA/iMe- dC/AAAGGAA | 34 |
| | 6-10 | | +A+G+G+C+AUoAGGATGA/iMe- dC/AAAGGAA | 35 |
| | 6-11 | | +A+G+GCoAo+TAGGATGA/iMe- dC/AAAGGAA | 36 |
| | 6-12 | | +A+G+G+CAoUoAGGATGA/iMe- dC/AAAGGAA | 37 |
| | 6-13 | | +A+G+GCo+AUoAGGATGA/iMe- dC/AAAGGGAA | 38 |
| | 6-14 | | +A+G+GCoAoUoAGGATGA/iMe- dC/AAAGGGAA | 39 |

### (1) Experimental method

An experiment was performed to analyze the expression level of miR-204 following treatment with Examples 6-1 to 6-14 through qRT-PCR using the same method as in Experimental Example 4 above. At this time, the statistical significance indicators in the graph are as follows.
*, **, ***, ****: Comparison with Vehicle (P < 0.05, 0.01, 0.001, 0.0001, respectively);
#, ##, ###, ####: In Examples 6-1 to 6-14, ASO (1 µM concentration group) was compared with Full DNA (P < 0.05, 0.01, 0.001, 0.0001, respectively);
$, $$, $$$, $$$$: In Examples 6-1 to 6-14, ASO (10 µM concentration group) was compared with Full DNA (P < 0.05, 0.01, 0.001, 0.0001, respectively).

### (2) Interpretation of experimental results

Regarding the efficacy of ASO against miR-204, experiments were performed to confirm the effect of introducing modified sugars other than LNA in the second region (nucleotide 4 to 7 based on ASO). To this end, ASOs including one to three MOE modifications at the corresponding positions (Examples 6-1 to 6-7) and ASOs including OME modifications at the same positions (Examples 6-8 to 6-14) were designed and tested under the same conditions as Example 1 above.

The qRT-PCR analysis results showed that the miR-204 degradation effect was maintained even when MOE or OME was introduced into the second region, and statistically significantly higher miR-204 degradation capability was confirmed compared to the control group (Full DNA) from which LNA was completely excluded (see FIGS. 12 and 13). These results confirmed that the efficacy of ASO was not significantly reduced even when modified sugars other than LNA were partially introduced into the second region.

These results suggest that the overall structural stability of the ASO and the miR-204 degradation activity may be maintained even when MOE or OME is introduced in replacement of LNA in the second region (positions 4 to 7), indicating that modified ASO design with secured chemical diversity is possible.

### [Experimental Example 9] Effects of miR-204-specific ASO in cellular models

The miR-204 degradation effect of Example 1 was confirmed in Experimental Example 1 above. Furthermore, to confirm whether the ASO of Example 1 also degrades miR-204 and exhibits effects for preventing or treating OA in cellular models, the following experiments were performed.

### (1) Experimental method

### 1) Primary culture of mouse chondrocyte

Mouse articular chondrocytes were isolated from the femoral condyles and tibial plateaus of 5 to 6 day-old ICR mice. The isolated tissues were enzymatically digested using a 0.2% collagenase solution to obtain a single-cell suspension. The obtained cells were inoculated and cultured (primary culture) in DMEM supplemented with 10% FBS (Gibco) and 1% penicillin-streptomycin (Sigma-Aldrich) at different cell densities according to each experimental purpose. The cells were maintained under hypoxic conditions at 37 °C, 5% CO₂, and 3% O₂. At 72 hours after cell inoculation, ASO treatment groups and vehicle control groups were established according to each experimental purpose. To induce senescence of chondrocytes, primary cultured chondrocytes were treated with bleomycin (Cayman) 40 µg/ml for 24 hours. Thereafter, the medium was removed and replaced with growth medium using NFW as vehicle or growth medium containing Example 1 (SEQ ID NO: 1) (final concentration 2 µM). At 48 hours after treatment, cells were harvested for mRNA analysis, with an inoculation density of 5 × 10⁴ cells/well in 12-well plates. In addition, at 72 hours after treatment with Example 1 (final concentrations 0.1, 1, 10 µM), cells were harvested for protein analysis, with an inoculation density of 2 × 10⁵ cells/well in 6-well plates. To induce an inflammatory response in chondrocytes, primary cultured chondrocytes were treated with IL-18 (Genscript) 1 ng/ml and simultaneously co-treated with Example 1 (10 µM) or SM04690 (30 nM) for 72 hours. Thereafter, cells were harvested for mRNA analysis, with an inoculation density of 0.83 × 10⁵ cells/well in 12-well plates.

### 2) Analysis of gene expression changes by Example 1 through qRT-PCR and Western blot

Cells in culture were harvested, and total RNA was extracted using TRI Reagent (Molecular Research Center, Inc.). The extracted RNA was synthesized into cDNA using SuperScript IV reverse transcriptase (Invitrogen). The synthesized cDNA was used for qRT-PCR using SYBR Green qPCR Master Mix (Thermo Fisher) to analyze the expression levels of chondrocyte ECM constituent-related genes and inflammation-related genes. The Hprt gene was used as an internal control, and the relative expression level of each gene was calculated as relative expression compared to the vehicle control group according to the ΔΔCt method. For statistical analysis, all quantitative data were expressed as Mean ± SEM. Statistical significance was test using GraphPad Prism 10 software (version 10.4.2), and Tukey's post-hoc test was applied after performing one-way ANOVA. The primers used for qRT-PCR are shown in Table 13 below.

**[Table 13]**

| **Target gene** | | **Sequence** | **NCBI sequence number** | **SEQ ID NO** |
|---|---|---|---|---|
| ***Chst11*** | Sense | 5'-AACCCAAGAAGAGAGCAGATG- 3' | NM_021439.4 | 41 |
| | Antisense | 5'-AGCCTGGAGAATAGTAGATGC- 3' | | 42 |
| ***Chsyl*** | Sense | 5'-TGGTCATTCTGCTTTTCAACTC- | NM_001081163.2 | 43 |
| | | 3' | | |
| | Antisense | 5'-ACTCGGTAATCTCGCATCAG-3' | | 44 |
| ***Haplnl*** | Sense | 5'-CAGGACAAGCAGAGAAGAGTC- 3' | NM_013500.4 | 45 |
| | Antisense | 5'-TCCTCCAGGGTAAGATCCGT-3' | | 46 |
| ***Has2*** | Sense | 5'- GCGTGGATTATGTACAGGTGTG-3' | NM_008216.3 | 47 |
| | Antisense | 5'-GTCTGCTGGTTCAGCCATCT-3' | | 48 |
| ***Hprtl*** | Sense | 5'-ATACAAAGCCTAAGATGAGCG- 3' | NM_013556.2 | 49 |
| | Antisense | 5'-GCAAATCAAAAGTCTGGGGA-3' | | 50 |
| ***Il-6*** | Sense | 5’- GGAGACTTCACAGAGGATACCA-3’ | NM_031168.2 | 51 |
| | Antisense | 5'-TCATTTCCACGATTTCCCAGAG- 3' | | 52 |
| ***Mmp 3*** | Sense | 5'-TTGATGGGCCTGGAACAGTC-3' | NM_010809.3 | 53 |
| | Antisense | 5'-AGTCCTGAGAGATTTGCGCC-3' | | 54 |
| ***Slc35d1*** | Sense | 5'-TGCTCACCAACTACAGATTTCC- 3' | NM_001356276.1 | 55 |
| | Antisense | 5'-GCTGCTGTCAGGACATCATTG- 3' | | 56 |

For protein expression analysis, cells in culture were harvested, and total protein was extracted using a radioimmunoprecipitation assay (RIPA) buffer. Quantified protein samples were separated by sodium dodecyl sulfate-polyacrylamide gel electrophoresis (SDS-PAGE) and then transferred to nitrocellulose (NC) membrane. Thereafter, primary and secondary antibody reactions were performed for cartilage matrix synthesis-related proteins (HPLN1, CHSY1) and internal control protein (vinculin). Protein signals were detected using SuperSignal West Dura Extended Duration Substrate Enhanced Chemiluminescence (ECL) solution (Thermo Fisher) and confirmed using a chemiluminescent signal detection system (iBright FL1500, Thermo Fisher). HPLN1 antibody (ab98038) was purchased from Abcam, CHSY1 antibody (NBP3-47604) was purchased from Novus Biologicals, and vinculin antibody (13901) was purchased from Cell Signaling Technology.

### 3) sGAG assay

sGAG quantitative analysis was performed using primary cultured mouse chondrocytes according to the above-described method. To induce cellular senescence, cells were treated with bleomycin at a concentration of 40 µg/mL for 24 hours. After treatment, the cells were further cultured for three days in fresh complete medium with or without Example 1. The medium was then replaced and cultured for another three days, after which the culture supernatant was collected and used for sGAG analysis. The concentration of sGAG in the collected culture medium was measured using 1,9-dimethylmethylene blue (DMMB) staining method. Absorbance was measured at 525 nm, and sGAG content was quantified based on a standard curve prepared using chondroitin sulfate (C0335; Tokyo Chemical Industry CO., LTD.). To correct for differences in cell viability and cell density, MTT assay was performed on the same cells. To this end, PBS-based MTT solution (3-(4,5-dimethylthiazol-2-yl)-2,5-diphenyltetrazolium bromide, T-030-1; Goldbio) was added at a concentration of 100 µg/mL and allowed to react for two hours. After the reaction, the generated formazan crystals were dissolved in dimethyl sulfoxide (DMSO), and absorbance was measured at 570 nm. All sGAG measurements were normalized based on the MTT results for comparison and analysis. The maximum effect (Eₘₐₓ) and half-maximal effective concentration (EC₅₀) of Example 1 were calculated using GraphPad Prism (Dotmatics).

### 4) Pathway Analysis

C28/I2 cell lines overexpressing miR-204 were treated with Example 1 at 1 µM concentration, and a differentially expressed gene list (DEG list) with significantly altered expression was derived from the obtained transcriptome data. Using the derived DEG list, reactome pathway analysis was performed based on the EnrichR platform. The analysis results selected the top 20 annotations based on adjusted p-value and were visualized and presented in bubble plot format.

### (2) Experimental results

Since miR-204 is present at a very low basal level in normal chondrocytes, a chondrogenic cell line (C28/I2) overexpressing miR-204 was used to sensitively evaluate the miR-204 degradation efficacy of Example 1. Transcriptome analysis confirmed that gene groups related to ECM remodeling, including ECM proteoglycans, and biological pathways (gene annotations) related to cartilage structure maintenance were significantly altered (FIG. 15). This suggests that the inhibition of miR-204 by Example 1 is not limited to simple microRNA-level regulation, but also involves structural repair mechanisms such as ECM composition and homeostasis maintenance within chondrocytes.

Based on the above-described results, the protective action mechanism of Example 1 was verified at the molecular level in primary cultured mouse chondrocytes. To this end, consistent with the results confirmed in the transcriptome analysis, changes in the expression of key enzymes in the sulfated proteoglycan biosynthesis pathway were analyzed. When chondrocytes damaged by bleomycin were treated with Example 1, the expression of key enzymes in the sGAG biosynthesis pathway increased statistically significantly, while the expression of matrix-degrading enzymes decreased significantly (FIG. 16A). This expression recovery indicates that the cartilage matrix synthesis pathway, which had been inhibited by miR-204, was restored to normal levels by Example 1, and the same increasing trend was confirmed at the protein level following Example 1 treatment (FIG. 16B). These results molecularly and functionally demonstrate that Example 1 effectively inhibits the pathological action of miR-204 while simultaneously restoring the matrix synthesis capacity of chondrocytes.

Next, to evaluate the efficacy of Example 1 under inflammatory stimulation conditions, experiments were performed using primary cultured chondrocytes treated with the inflammatory cytokine IL-1β. As a result, similar to the results observed when damage was induced with bleomycin, the expression of proteoglycan biosynthetic enzymes was significantly restored in the Example 1 treatment group, while the expression of the inflammatory cytokine IL-6 was statistically significantly decreased (FIG. 16C). Notably, Example 1 showed superior effects compared to SM04690, which has completed a phase III clinical trial.

These results demonstrate that Example 1 effectively inhibits the miR-204-mediated pathway not only in senescent environments but also in inflammatory environments, thereby performing dual action of promoting cartilage matrix synthesis and alleviating inflammatory responses.

Next, additional experiments were carried out to verify that the effect of Example 1 is not limited to changes at the gene and protein levels, but also affects the functional expression of actual cells. As a result of the treatment of Example 1 in senescence-induced chondrocytes, it was confirmed that the sGAG content was recovered in a concentration-dependent manner (FIG. 17A). Quantitative analysis showed that the maximum effect (Eₘₐₓ) of Example 1 was 114.3%, and the half-maximum active concentration (EC₅₀) was 273.5 nM (FIG. 17B). Furthermore, an increase in the sGAG content by Example 1 was confirmed even in an inflammatory environment, and its effect was superior to that of SM04690 (FIG. 17C).

Therefore, Example 1 was proven to be a therapeutic candidate substance that structurally and functionally restores the ECM synthesis function of chondrocytes by effectively blocking the pathological signaling pathway of miR-204 in both senescent and inflammatory environments.

### [Experimental Example 10] Confirmation of effect of miR-204-specific ASO in animal model

The miR-204 degradation effect of Example 1 was confirmed in Experimental Example 1 above. Furthermore, to confirm whether the ASO of Example 1 also degrades miR-204 in an animal model and exhibits an effect of preventing or treating OA, the following experiments were performed.

### (1) Experimental methods

### 1) Construction of OA mouse model

C57BL/6 male mice (12 weeks old, WT) used in the experiments were purchased from Daehan Biolink Co. LTD., Eumsung, Korea. All animal experiments were approved by the Institutional Animal Care and Use Committee (IACUC) of Seoul National University (SNU-240710-5-1). Laboratory animals were randomly assigned and housed in a specific pathogen-free (SPF) facility at Seoul National University. Housing conditions were maintained at a temperature of 23 to 25 °C, a humidity of 45% to 65%, and a 12-hour light/dark cycle. All mice were provided with standard laboratory chow *ad libitum.* Animal experiment reporting complied with the Animal Research: Reporting of In Vivo Experiments (ARRIVE) guidelines. To induce surgical OA (post-traumatic OA), destabilization of the medial meniscus (DMM) surgery was performed on the right knee of 12-week-old male mice, with sham surgery performed under identical conditions as a control.

To evaluate the therapeutic efficacy of Example 1 for OA, Example 1 and SM04690 were each administered intra-articularly into the knee joint at week 4 post-surgery, while dexamethasone was administered once weekly to the same site starting from week 2 post-surgery. Mice were sacrificed at week 6 post-administration for histological analysis. In addition, to evaluate the therapeutic effect in late-stage OA, Example 1 was administered intra-articularly twice at 2-week intervals starting from week 4 post-surgery, and mice were sacrificed at week 4 after the final administration for histological analysis. Static weight-bearing analysis was performed one day prior to sacrifice.

### 2) Histological analysis, immunohistochemistry, and in situ hybridization

For histological analysis, legs of sacrificed mice were excised and fixed in 4% paraformaldehyde solution at 4 °C. The fixed leg tissues were decalcified in 0.5 M ethylenediaminetetraacetic acid (EDTA) solution (pH 7.4) at 4 °C for four weeks, then sequentially dehydrated with ethanol, treated with xylene, and embedded in paraffin. The prepared paraffin blocks were sectioned at 5 µm thickness to prepare slides.

The slides were deparaffinized with xylene and then rehydrated with ethanol in stepwise decreasing concentrations. Thereafter, safranin O staining was performed to observe changes in cartilage tissue, and the degree of damage to the medial tibial cartilage was evaluated in a double-blind manner according to the Osteoarthritis Research Society International (OARSI) criteria (grades 0 to 6).

Immunohistochemistry staining was performed using SLC35D1 (ab113717, Abcam), IL6 (sc-130326, Santa Cruz Biotechnology, Inc.), and CTXII (NBP2-59386, Novus Biologicals) antibodies diluted at 1:100, and CHSY1 (NBP3-47604, Novus Biologicals), HPLN1 (ab98038, Abcam), and HAS2 (NBP3-03787, Novus Biologicals) antibodies diluted at 1:50.

*In situ* hybridization was performed using a probe for the detection of miR-204 and a probe for the detection of Example 1 (manufactured by BIONEER Inc.), with a U6 snRNA detection probe purchased from Exiqon. The tissue was treated with 5 µg/ml proteinase K at 37 °C for 30 minutes and then re-fixed with 4% paraformaldehyde solution at room temperature for 20 minutes. The hybridization reaction was carried out under the following conditions: U6 snRNA probe (1 nM) was allowed to react at 50 °C for one hour, Example 1 probe (50 nM) was allowed to react at 42 °C for two hours, and miR-204 probe (50 nM) was allowed to react at 36 °C for two hours. Thereafter, anti-digoxigenin (DIG)-alkaline phosphatase (AP) antibody (1:800 dilution, Roche, 11093274910) was allowed to react at room temperature for one hour, and the signal was developed using a nitro blue tetrazolium/5-bromo-4-chloro-3-indolyl phosphate (NBT/BCIP) solution (Thermo Fisher, 34070).

All histological, immunohistochemical, and *in situ* hybridization images were acquired using a Nikon Eclipse Ni-U optical microscope equipped with a DS-Ri2 camera (Nikon).

### 3) Micro-computed tomography (µCT) analysis

To evaluate the therapeutic effect of Example 1 and potential side effects on bone remodeling, the knee joints of mice were excised and fixed by the above-described method. The therapeutic effect evaluation was performed using a Bruker SKYSCAN 1276 *in vivo* µCT system (70 kV, 57 µA, 0.5 mm Al filter, imaging time 13 minutes), and the obtained images were reconstructed using NRecon software (Bruker, version 1.7.3.2). Three-dimensional images were generated using CTvox (Bruker, version 3.3.0).

### (2) Interpretation of Experimental Results

The therapeutic efficacy of Example 1 was evaluated in a surgically induced OA mouse model. In the DMM surgical group injected with a vehicle (administration control group), typical pathological changes of OA such as cartilage destruction, subchondral bone sclerosis, osteophyte development, and synovial inflammation were severely manifested. In contrast, in the group administered Example 1, DMM-induced cartilage damage was statistically significantly suppressed, and the degree of subchondral bone sclerosis, osteophyte maturation, and synovial inflammation was also significantly alleviated (FIGS. 18A to 18C). *In situ* hybridization analysis confirmed that Example 1 persistently remained in the articular cartilage at doses of 10 µg or higher even six weeks after administration (FIG. 18D), suggesting that Example 1 can stably reside in cartilage for extended periods and exert sustained therapeutic effects. In behavioral analysis, intra-articular administration of Example 1 statistically significantly restored the weight-bearing imbalance caused by DMM surgery, indicating that this drug can substantially alleviate OA-associated pain behavior (FIG. 18E). Analysis at the molecular level showed that the Example 1 treatment group exhibited restored expression of enzymes involved in chondroitin sulfate synthesis and significantly reduced expression of proteins associated with cartilage matrix destruction (FIG. 18F), demonstrating contribution to structural recovery and functional restoration of cartilage tissue.

The efficacy of Example 1 was consistently maintained in repeated administration experiments. Even when the ASO was injected twice, both cartilage damage and subchondral bone sclerosis were markedly reduced, and the pain alleviation effect was also sustained. Particularly, the overall efficacy of Example 1 was superior compared to the steric blocker (Comparative Example 4) (SEQ ID NO: 22) under the same conditions, showing stable and reproducible effects even after repeated administration (FIGS. 19A to 19D).

To verify the therapeutic efficacy of Example 1, comparative experiments were conducted with drugs currently in clinical use or under development. Experiments were performed using as controls SM04690, an OA therapeutic agent that completed a phase 3 clinical trial, and dexamethasone, a steroidal antiinflammatory analgesic, and the results showed that SM04690 exhibited some pathological improvement effects but had limited cartilage protective effects compared to Example 1, and dexamethasone was also confirmed to have antiinflammatory and pain alleviation effects but exhibited low cartilage regeneration capacity. On the other hand, the Example 1 administration group showed the most significant reduction in cartilage destruction and exhibited the best subchondral bone sclerosis amelioration and pain alleviation effects (FIGS. 20A to 20E). These results suggest that unlike conventional inflammation control-centered drugs, Example 1 ameliorates the pathophysiological sources by inducing protection and restoration of the cartilage structure itself.

In addition, to evaluate the therapeutic potential in late-stage OA, when drugs were administered six weeks after OA induction, statistically significant cartilage recovery was observed only in the Example 1 administration group, while no significant effects were confirmed in the steric blocker (Comparative Example 4) (SEQ ID NO: 22) and SM04690 administration groups (FIGS. 21A to 21C). However, pain alleviation effects were confirmed at statistically significant levels in all three groups: Example 1, steric blocker (Comparative Example 4) (SEQ ID NO: 22), and SM04690 (FIG. 21D). Micro-CT analysis also showed that Example 1 most effectively alleviated subchondral bone sclerosis, providing mechanistic evidence for restoring structural stability of subchondral bone.

In summarizing the above-described results, it was confirmed that Example 1 is an excellent therapeutic candidate that inhibits the pathological progression of OA at multiple levels compared to other drugs. This substance demonstrated outstanding therapeutic effects in structural, functional, and behavioral aspects through a complex mechanism of action, including: (1) inhibition of cartilage destruction and promotion of regeneration, (2) reduction of subchondral bone sclerosis and osteophyte development, (3) alleviation of synovial inflammation and suppression of inflammatory responses, (4) pain amelioration and functional recovery, and (5) maintenance of long-term efficacy through sustained retention within cartilage. In particular, Example 1 exhibited superior cartilage protection and pain alleviation effects compared to existing clinical-stage drugs or steroids and maintained reproducible efficacy in both single and repeated administrations. These results strongly suggest that Example 1 is not merely a symptomatic relief agent, but a mechanism-based ASO drug candidate capable of ameliorating the fundamental pathophysiology of OA.

### [Experimental Example 11] Confirmation of off-target effects of Example 1

The miR-204 degradation effect of Example 1 was confirmed in Experimental Example 1 above. Furthermore, to evaluate the off-target effects of the ASO of Example 1, the following experiments were performed.

### (1) Experimental methods

### 1) Predicted target analysis of Example 1

Predicted target genes (human mRNA, microRNA, and pre-mRNA) were analyzed for sequence variants including the nucleotide sequence (n) of Example 1, a form with one nucleotide truncated from the 3' end (n-1), and a form with one nucleotide repeated and added at the 3' end (n+1). Sequence-based homology analysis was performed using NCBI-BLAST, and human gene databases provided by NCBI (human RefSeq_RNA and human RefSeq_Gene) were used for the analysis. In evaluating sequence alignment, cases with two or fewer mismatched bases were set as the criterion for significant homology.

### 2) GO term analysis

A DEG list was derived from transcriptome data obtained after treating C28/I2 cell lines overexpressing miR-204 with Example 1 at a concentration of 1 µM. GO term analysis was performed using the derived DEG list based on the EnrichR platform. For the analysis results, the top 10 annotations were selected based on adjusted p-value and visualized in bubble plot format.

### (2) Interpretation of experimental results

Predicted target genes (human mRNA, microRNA, and pre-mRNA) capable of binding were analyzed based on the nucleotide sequence of Example 1. Homology evaluation using NCBI-BLAST revealed that no sequences expected to bind were identified except for TRPM3, which is the host gene of miR-204, and the on-target miR-204 (see Table 14). Table 14 shows the results of sequence homology analysis performed using NCBI-BLAST on the nucleotide sequence and sequence variants of Example 1.

**[Table 14]**

| Number of nucleotides in sequence | Human mRNA or microRNA | Human pre-mRNA |
|---|---|---|
| | NA | *TRPM3* |
| n-1 | NA | *TRPM3* |
| n+1 | NA | *TRPM3* |

| | | |
|---|---|---|
| *TRPM3 is the host gene of miR-204. | | |

In addition, GO analysis was performed using transcriptome data obtained after treating C28/I2 cell lines overexpressing miR-204 with Example 1. The analysis was conducted using EnrichR, and the top 10 annotations were selected based on adjusted p-value. As a result, no significant ontology terms were derived in any of the Biological Process, Molecular Function, or Cellular Component categories (see FIGS. 22A to 22C). These results suggest that Example 1 is a highly specific ASO with high miR-204 sequence specificity and extremely low off-target effects.

### [Experimental Example 12] Confirmation of immunogenicity of Example 1

The miR-204 degradation effect of Example 1 was confirmed in Experimental Example 1 above. Furthermore, to confirm the immunogenicity of Example 1, the following experiments were performed.

### (1) Experimental method - TLR activity assay

To evaluate the effect of Example 1 on TLR activity, a TLR activity assay was performed using HEK-Blue cell lines (InvivoGen) overexpressing each of TLR7, TLR8 and TLR9. HEK-Blue TLR7 cell lines were plated into 96-well plates at a density of 5 × 10⁴ cells/well and cultured for 24 hours. Thereafter, Example 1 (10 µM) or ORN6S (positive control) was treated together with 0.1 mM guanosine (Sigma, G6264). After six hours of drug treatment, the cell culture medium was collected, and secreted alkaline phosphatase (SEAP) activity in the culture supernatant was measured. HEK-Blue TLR8 cell lines were inoculated at the same density and cultured for 24 hours, and then treated with Example 1 (10 µM) or ORN6S together with 1 mM uridine (Sigma, U3003). After six hours of treatment, the culture supernatant was collected, and SEAP activity was measured. HEK-Blue TLR9 cell lines were cultured under the same conditions, and then treated with Example 1 (10 µM) or ODN2006 (positive control). After six hours of treatment, the cell culture medium was collected, and SEAP activity was measured.

### (2) Interpretation of experimental results

To evaluate the immunogenicity of Example 1, changes in the activity of TLRs 7, 8, and 9 were measured. As a result of analyzing ligand-dependent SEAP expression using HEK-Blue TLR7, TLR8, and TLR9 cell lines, ORN6S (for TLR7 and TLR8 assays) and ODN2006 (for TLR9 assay), used as positive controls, both induced receptor activity, resulting in a significant increase in SEAP activity.

On the other hand, no significant increase in TLR7, TLR8, or TLR9 activity was observed under any conditions treated with Example 1 (10 µM) (see FIGS. 23A to 23C). These results indicate that Example 1 does not induce an immune response through TLR7, TLR8, or TLR9 and has non-immunostimulatory properties for the innate immune receptor pathway. Therefore, the results suggest that Example 1 is a safe ASO with low potential for immunogenicity induction.

### [Experimental Example 13] Confirmation of toxicity of Example 1

The miR-204 degradation effect of Example 1 was confirmed in Experimental Example 1 above. Furthermore, since it is necessary to examine the safety when utilizing the composition including Example 1 as a pharmaceutical composition, the following experiments were performed to confirm the toxicity of Example 1.

### (1) Experimental method

### 1) Cytotoxicity assay

To evaluate the cytotoxicity of Example 1, mouse primary chondrocytes, a hepatocyte cell line (HepG2 cell line), and a renal cell line (RPTEC cell line) were used. The HepG2 and RPTEC cell lines were purchased from the American Type Culture Collection (ATCC).

Each cell line was plated into a 96-well plate under the following conditions: mouse primary chondrocytes 1 × 10⁴ cells/well; HepG2 cells 1 × 10³ cells/well; and RPTEC cells: 4 × 10⁴ cells/well. After culturing each cell line for 72 hours (chondrocytes) or 24 hours (HepG2 and RPTEC cells) under respective conditions, Example 1 was treated at concentrations of 2 µM, 5 µM, and 10 µM.

Seventy two hours after treatment, cell viability was measured using the CellTiter-Glo^{®} Luminescent Cell Viability Assay Kit (Promega).

### 2) Single-dose toxicity studies in mice

To evaluate the toxicological properties of Example 1, two single-dose toxicity studies were performed. First, a 2-week single-dose toxicity study was conducted using 9-week-old female CD-1 mice (Daehan Biolink CO., LTD., Eumsung, Korea). This study was approved by the Seoul National University IACUC (approval number: SNU-230531-6-1). The animals were housed in an SPF animal laboratory at Seoul National University, where they were provided with standard laboratory chow *ad libitum* under conditions of at a temperature of 23 to 25 °C, a relative humidity of 45% to 65%, and a 12-hour light/dark cycle. The mice were randomly assigned to three groups, each receiving a single intra-articular injection of 5 µL NFW including Example 1 (20 µg, 40 µg, and 80 µg, respectively). Body weight was measured on the day of administration (day 0), day 4, day 11, and day 14. On day 14 post-administration, blood was collected through the retro-orbital sinus after one day of fasting, and plasma was separated by centrifugation (1,500 × g, 15 min, 4 °C). Clinical chemistry analysis was performed by an external laboratory (CellPurics Inc., Korea) using an Adivia 1800 automated analyzer (Siemens). After sacrifice, major organs (liver, spleen, kidneys) were removed, weighed, and examined macroscopically for abnormalities. Right leg tissue was excised and fixed in 4% paraformaldehyde solution at 4 °C overnight, and cartilage and bone tissues were analyzed for abnormalities by histological analysis using the above-described method. Chondrocyte apoptosis was assessed using TUNEL assay (Takara, MK500) according to the manufacturer's instructions, and nuclei were stained with DAPI for 10 minutes at room temperature. Fluorescence signals were observed using an EVOS M7000 Imaging System (Thermo Fisher, AMF7000).

### (2) Interpretation of Experimental Results

The cytotoxicity of Example 1 was confirmed in mouse cartilage-derived primary cultured cells, hepatocyte cell line (HepG2 cell line), and renal cell line (RPTEC cell line). As a result of the experiment, no significant level of cytotoxicity that reduced cell viability to 80% or below was observed in any of the three cell types (see FIGS. 24A, 24B, and 24C). Next, to evaluate the local and systemic safety of Example 1, a 14-day toxicity study was conducted in mice following single-dose intra-articular (IA) administration. Example 1 was administered at doses of 20, 40, and 80 µg/animal. As a result, the organ-to-body weight ratios of liver, kidneys, and spleen showed no significant differences from the control group in any treatment groups (see FIG. 25A). This suggests that no systemic or organ-specific toxicity occurred following the administration of Example 1. Histological analysis of the administration site revealed no cartilage damage or proteoglycan loss within the knee joint tissue, and safranin-O staining confirmed that the matrix components within the cartilage were maintained normally (FIG. 25B, upper and middle panels). Additionally, TUNEL staining analysis confirmed that chondrocyte apoptosis was not induced, thereby demonstrating the excellent local tissue tolerability of Example 1 (FIG. 25B, lower panel). The above-described results provide evidence that Example 1 is a safe ASO that exhibits both excellent local safety and absence of systemic toxicity when administered intra-articularly.

### [Experiment 14] Effect of miR-204-specific ASO on canine chondrocytes

The miR-204 degradation effect of Example 1 was confirmed in Experimental Example 1 above. Furthermore, to verify whether the ASO of Example 1 can effectively inhibit miR-204 in canine chondrocytes, the following experiments were performed.

### (1) Experimental methods

### 1) Canine chondrocytes cell line culture method

A canine chondrocyte cell line (Cell Applications, Inc.) was plated into 12-well plates at a density of 2×10⁵ cells/well and cultured for 24 hours. Cells were maintained at 37°C under 5% CO₂ and atmospheric oxygen concentration conditions, and the culture medium used was canine chondrocyte growth medium (Cell Applications, Inc., Cn411-500) supplemented with growth supplement (Cell Applications, Inc., Cn411-GS). After 24 hours of culture, cells were used according to each experiment.

### 2) Confirmation of Example 1 delivery efficiency

In order to visualize the intracellular uptake pattern of Example 1 into canine chondrocytes, the cells during culture were cultured with Cy3 fluorescent marker Example 1 at a concentration of 1µM for 24 hours. Thereafter, co-incubation was performed for 20 minutes by adding Hoechst at a concentration of 1 µg/ml. For fluorescent microscopy analysis, the cells were washed three times with PBS, and fluorescent images were obtained using the EVOS M7000 Imaging System (Thermo-Fisher, AMF7000).

In addition, in order to quantitatively evaluate the level of intracellular uptake by flow cytometry, canine chondrocytes were cultured with Cy3 fluorescent marker Example 1 (10 nM or 50 nM) for 24 h. After treatment, the cells were separated from the attachment surface with trypsin, washed with PBS, centrifuged and recovered. The recovered cells were resuspended twice in a PBS solution containing 5% FBS.

Fluid cell analysis was performed using FACSAria^{™} III cell sorter (BD Biosciences), and analytical data was collected and quantitatively analyzed using FACSDiva 9.7 software.

### 3) qRT-PCR

Canine chondrocytes were cultured for 24 hours and treated with 0.25 µM doxorubicin 0.25 µM (Sigma-Aldrich) to induce cell senescence. The cells were maintained under hypoxic conditions of 37 °C, 5% CO₂ and 3% O₂. After 72 hours, the cells were treated again with the same concentration of doxorubicin, but half of the existing culture solution was removed, and the remaining half was diluted with the same volume of fresh culture solution.

The cells were recovered, and total RNA was extracted using TRI reagent (Molecular Research Center, Inc.). The extracted RNA was synthesized into DNA using miRCURY LNA RT kit (Qiagen N.V.). The synthesized cDNA was analyzed for miR-204 expression by quantitative reverse polymerase chain reaction (qRT-PCR) using miRCURY LNA microRNA PCR Kit (Qiagen N.V.).

### 4) sGAG assay

sGAG quantitative analysis was performed using primary cultured canine-chondrocytes according to the above-described method. To induce cellular senescence, cells were treated with doxorubicin (Sigma-Aldrich) at a concentration of 0.25 µM. The cells were maintained under hypoxic conditions at 37 °C, 5% CO₂, and 3% O₂. After 72 hours, cells were treated with doxorubicin again at the same concentration. However, half of the existing culture medium was removed, and the remaining half was mixed with the same volume of fresh culture solution for dilution before treatment. After an additional 72 hours had elapsed, cells were treated with Example 1 at concentrations of 0.1, 1, and 10 µM while replacing with fresh culture solution, and the medium was replaced with fresh medium 48 hours after treatment. After culturing for an additional two days, the culture supernatant (culture medium) was collected and used for sGAG analysis. The concentration of sGAG in the collected culture solution was measured using DMMB staining. Absorbance was measured at 525 nm, and sGAG content was quantified based on a standard curve prepared using chondroitin sulfate (C0335; Tokyo Chemical Industry CO., LTD.). To correct for differences in cell viability and cell density, an MTT assay was performed on the same cells. To this end, a PBS-based MTT solution (T-030-1; Gold Biotechnology, Inc.) was added at a concentration of 100 µg/mL and allowed to react for two hours. After the reaction, the generated formazan crystals were dissolved in DMSO, and absorbance was measured at 570 nm. All sGAG measurements were normalized based on MTT results for comparative analysis. Statistical significance was test using GraphPad Prism 10 software (GraphPad Software), and Tukey's post-hoc test was applied after performing one-way ANOVA.

### (2) Interpretation of experimental results

To confirm the delivery efficiency of Example 1 to canine chondrocytes, intracellular uptake was evaluated using Cy3 fluorescently-labeled Example 1. Fluorescence microscopy analysis confirmed that fluorescent signals were distinctly accumulated intracellularly in canine chondrocytes of the Example 1 treatment group compared to the vehicle treatment group (FIG. 26A). Furthermore, flow cytometry analysis revealed that fluorescent positive signals of more than 50% were observed under all conditions when cells were treated with Example 1 at concentrations of 10 nM and 50 nM, quantitatively confirming the excellent intracellular uptake capability of Example 1 (FIG. 26B). These results suggest that Example 1 has physicochemical properties that enable efficient delivery into chondrocytes and stable accumulation.

Thereafter, the reduction of miR-204 by Example 1 was confirmed in canine chondrocytes with induced cellular senescence. After treating with doxorubicin to induce cellular senescence, a statistically significant decrease in miR-204 expression was confirmed when Example 1 was delivered (FIG. 26C).

Subsequently, the effect of Example 1 on the recovery of synthetic function of cartilage matrix components was evaluated. When canine chondrocytes induced cellular senescence were treated with Example 1, it was confirmed that the synthesis of sGAG, which had decreased due to senescence, was significantly increased (FIG. 26D). These results indicate that Example 1 has functional efficacy in restoring ECM synthesis function through miR-204 degradation not only in mouse chondrocytes but also in canine chondrocytes, supporting matrix recovery and anti-aging effects at the cellular level.

These results suggest that Example 1 has cross-species efficacy in mammals such as dogs and cats, being capable of effectively inhibiting miR-204 not only in mouse-derived chondrocytes but also in canine chondrocytes, and support the conservation of the miR-204 target inhibition mechanism regardless of the species specificity of the substance.

## Claims

1. An oligonucleotide specifically hybridizing with miR-204,
wherein the oligonucleotide includes 20 to 22 linked nucleotides and consists of a first region, a second region, and a third region in order from a 5' end to a 3' end,
wherein the first region includes three consecutively connected nucleotides, each complementary to a tail region of miR-204 and including a constrained sugar modification,
the second region includes three connected nucleotides connected, the nucleotides included in the second region each independently being nucleotides including a constrained sugar modification or a non-constrained 2'-substituted sugar modification, and
the third region includes a plurality of connected nucleotides, the nucleotides included in the third region each independently being nucleotides including a non-constrained 2'-substituted sugar modification, and
wherein the oligonucleotide includes 30% or less of nucleotides including a constrained sugar modification based on a total number of nucleotides.

2. The oligonucleotide of claim 1, wherein the nucleotide including the constrained sugar modification is at least one selected from the group consisting of a constrained nucleic acid (LNA), a 2',4'-ethylene-bridged nucleic acid (ENA), constrained ethyl nucleotide (cEt), a bridged nucleic acid (BNA), and an acyclic amino-bridged BNA (BNANC).

3. The oligonucleotide of claim 1 or 2, wherein the non-constrained 2'-substituted sugar modification is at least one selected from the group consisting of 2'-deoxy, 2'-O-methyl (2'-O-Me), 2'-O-methoxyethyl (2'-O-MOE), 2'-O-alkyl, 2'-fluoro (2'-F), 2'-amino (2'-NH₂), 2'-O-allyl, 2'-O-benzyl, ribose, and arabinose (ANA) non-constrained 2'-substituted sugar modifications.

4. The oligonucleotide of any one of claims 1 to 3, wherein the nucleotides included in the third region all include a 2'-deoxy sugar.

5. The oligonucleotide of any one of claims 1 to 4, wherein sugars of the nucleotides at positions 1 and 2 from the 3' end of the oligonucleotide are 2'-deoxy.

6. The oligonucleotide of any one of claims 1 to 5, wherein the oligonucleotide includes a region complementary to a seed region of the miR-204, and sugars of nucleotides included in the region complementary to the seed region are non-constrained sugar modifications.

7. The oligonucleotide of any one of claims 1 to 6, wherein internucleotide bonds included in the oligonucleotide are each independently a phosphorothioate bond or a phosphodiester bond.

8. The oligonucleotide of any one of claims 1 to 7, wherein the oligonucleotide is one or more of oligonucleotides represented by SEQ ID NOs: 1 to 14 and 26 to 39.

9. The oligonucleotide of any one of claims 1 to 8, wherein the second region or the third region includes one or more mismatch nucleotides with the miR-204.

10. The oligonucleotide of any one of claims 1 to 9, wherein the oligonucleotide is an oligonucleotide for reducing and/or degrading the miR-204.

11. A pharmaceutical composition comprising an oligonucleotide of any one of claims 1 to 10 and a pharmaceutically acceptable carrier, vehicle or diluent.

12. The oligonucleotide according to any one of claims 1 to 10 for use as a medicament.

13. The oligonucleotide according to any one of claims 1 to 10 for use in a method of ameliorating, preventing, or treating osteoarthritis in a subject, preferably wherein the subject is a mammal.

14. The oligonucleotide for use according to claim 13, wherein the osteoarthritis is at least one selected from the group consisting of degenerative osteoarthritis, post-traumatic osteoarthritis, post-surgical osteoarthritis, inflammatory osteoarthritis, and secondary osteoarthritis.
